# EUROPEAN PATENT APPLICATION

(11) **EP 4 082 562 A2**
(43) Date of publication of application: **02.11.2022**
(21) Application number: 20905192.9
(22) Date of filing: 21.12.2020
(51) Int. Cl.: A61K 39/00, A61K 38/00, A61P 37/06, C07K 14/475

(54) **POLYPEPTIDES COMPRISING MUTATED FORMS OF HUMAN VEGF-A WITH REARRANGEMENTS OF DISULFIDE BONDS AND COMPOSITIONS CONTAINING SAME**

(30) Priority: 24.12.2019 CU 20190111
(71) Applicant: Centro De Ingenieria Genetica Y Biotecnologia, La Habana 11600 (CU)
(72) Inventor: BEQUET ROMERO, Mónica, La Habana 10400 (CU); MORERA DÍAZ, Yanelys, La Habana 11600 (CU); AYALA ÁVILA, Marta, La Habana 11600 (CU); GAVILONDO COWLEY, Jorge Victor, La Habana 10400 (CU); SÁNCHEZ RAMÍREZ, Javier, La Habana 10400 (CU); HERNÁNDEZ BERNAL, Francisco, La Habana 11600 (CU); GONZALEZ BLANCO, Sonia, La Habana 11600 (CU); ESPINOSA RODRÍGUEZ, Luis Ariel, La Habana 11400 (CU); BESADA PÉREZ, Vladimir Armando, La Habana 11600 (CU); PÉREZ DE LA IGLESIA, Mariela, La Habana 11700 (CU); TRIMIÑO LORENZO, Lian, La Habana 13400 (CU); LIMONTA FERNÁNDEZ, Miladys, La Habana 11300 (CU); UBIETA GÓMEZ, Raimundo, La Habana 11600 (CU)
(74) Representative: V.O.
(86) International application number: PCT/CU2020/050011
(87) International publication number: WO 2021/129898

(57) **Abstract**

Polypeptides comprising functional mutants of an isoform of the human vascular endothelial growth factor A (VEGF-A) folded in a non-natural re-arrangement, where the second and fourth cysteine of the mutant's polypeptide chain is only forming intramolecular bridges, while the seventh and eight are only part of intermolecular bonds. The invention further comprises antigenic preparations containing at least one of these polypeptides, and the pharmaceutical compositions comprising such antigenic preparations and vaccine adjuvants. The antigenic preparations according to the invention are used in the manufacturing of a drug, for the treatment of diseases related to the increment of angiogenesis, inflammation, and immunosuppression, as well as for the restoration of the immune system.

## Description

### Technical field

The invention relates to the fields of biotechnology and human health. It provides polypeptides comprising functional mutants of the human vascular endothelial growth factor A (VEGF-A), folded in a non-natural arrangement of disulfide bridges. The invention offers the basis for the generation of compositions comprising these polypeptides, which are used for the prophylaxes and therapeutics of pathologies advancing through the increment of angiogenesis, inflammation, and immunosuppression.

### Background of the invention

The VEGF-A system and its receptors are a molecular complex and their interactions specifically modulate the growth, permeability, plasticity, and motility of the endothelial cells, with a positive effect in pathological angiogenesis. Dysregulation of VEGF-A expression and their receptors occurs both in tumoral cells and the stroma, where they are particularly expressed and regulates the function of endothelial cells. The paracrine effects of tumoral VEGF-A on the endothelial cells surrounding the tumor, and the autocrine effects of this growth factor on the tumoral cells and the stroma expressing their receptors were mainly described in the last 20 years (Carmeliet, Nat Med, 2003: 9: 653-60, Mashreghi, et al., J Cell Physiol, 2018: 233: 2949-65).

VEGF-A induces in endothelial cells an increase in proliferation, motility, and organization resulting in the formation of new blood vessels with an arrangement and maturation that depend on the VEGF-A and it's isoforms concentration gradients as well as on the presence of other proangiogenic factors and their receptors (Carmeliet, Nat Med, 2003: 9: 653-60). Besides this primary function, the pieces of evidence on the expression of type 1 and 2 VEGF-A receptors and co-receptors in cells of myeloid origin, point to this factor as a principal mediator on inflammatory and immunosuppressive processes. It is known, for instance, that VEGF-A interacts with its type 1 receptor in dendritic cells and reduce their maturation via NFκB (Gabrilovich, et al., Nat Med, 1996: 2: 1096-103; Gabrilovich, et al., Blood, 1998: 92: 4150-66). Furthermore, VEGF-A binds to its type-2 receptor (VEGFR2), which is induced on effector T cells, as a control mechanism to inhibit interferon-gamma (IFN-γ) secretion (Ziogas, et al., Int J Cancer, 2012: 130: 857-64). VEGF-A is also an essential mediator of the inflammatory processes and induces the phenotypical change in macrophages and neutrophils at the microenvironment of lesions associated with neoplastic and arthritic phenomena among others (Voron, et al., Front Oncol, 2014: 4: 70). Within this context, the VEGF-A protein family and their receptors are a target for active and passive immunotherapy in diseases that progress through the already described process. The use of VEGF-A and their receptors as a target was validated by the acceptance of the inclusion of passive immunotherapies with Avastin, Sorafenib, and Sunitinib, as the first line of treatment for multiple tumor types, as well as for the age-related macular degeneration (AMD) (Wentink, et al., Biochim Biophys Acta, 2015: 1855: 155-71). The active immunotherapy with this growth factor, which implicates the induction of an autoimmune response, experience a slower development. Till 2002 only a few studies have been reported using VEGF-VEGFR as a target. Most of these efforts were dedicated to the study of the immunogenicity, anti-angiogenic and anti-tumoral effects of xenogenic variants (Wei, et al., Proc Natl Acad Sci USA, 2001: 98: 11545-50), or proteins with a high structural and functional homology to VEGFA (Patent Applications No. WO 99/45018 and WO 00/53219). While the use of xenogeneic variants induces high neutralizing and specific antibody titers in absence of cellular response, the use of homologous molecules results in a marginal immune response without shreds of evidence of anti-tumoral or anti-metastatic effects. Any of these strategies was translated into clinical practice.

The use of functional mutants of VEGF-A in the active immunization specific for this factor was described in 2002, either using naked deoxyribonucleic acid (DNA) or recombinant proteins fused to immunostimulatory sequences used as adjuvants (Patent Application No. PCT/CU03/00004).

The administration of VEGF-A mutants (Arg82, Lys84, His86 →Ala82, Ala84, Ala86; ο Arg82, Lys84, His86 →Glu82, Glu84, Glu86) result in the induction of a T-specific antitumoral response when using a naked DNA based immunization (Bequet-Romero, et al., Angiogenesis, 2007: 10: 23-34)), or a protein plus adjuvant based (Morera, et al., Angiogenesis, 2008: 11: 381-93). With the protein-based formulation, an induction of VEGF-A specific antibodies that inhibits its binding to VEGFR1 and VEGFR2 was achieved (Morera, et al., Angiogenesis, 2008: 11: 381-93, Morera, et al., Vaccine, 2012: 30: 368-77). Despite the mutation introduced to VEGF-A that hampers the protein binding to VEGFR-2, it was possible to induce antibodies that neutralize this interaction. The use of this vaccine strategy results in relevant antitumoral and antimetastatic effects in murine models of melanoma (MB16F10), lung carcinoma (3LLD122 y TC1), breast carcinoma (F3II), and colorectal carcinoma (CT26). For this variant a direct cellular response was demonstrated on syngeneic tumoral cells, accompanied by the secretion of IFN-γ after the incubation with VEGF-A) (Bequet-Romero, et al., Vaccine, 2012: 30: 1790-9). Differing from other strategies, this one uses a recombinant antigen produced in bacteria which is representative of VEGF-A isoform 121, where the cysteine residues involved in the formation of the VEGF-A dimer are not removed. The use of this strategy in studied species demonstrated the existence of a potential reserve of a superior immune response (Sanchez Ramirez, et al., BMC Immunol, 2017: 18: 39).

Using strategies like adjuvant changes and their concentrations, and also modifying the amount of antigen administered, higher immune responses were achieved with concomitant benefits in preclinical models and the clinics (Morera, et al., Angiogenesis, 2008: 11: 381-93; Gavilondo, et al., Vaccine, 2014: 32: 2241-50; Perez Sanchez, et al., Hum Vaccin Immunother, 2015: 11: 2030-7; Sanchez Ramirez, et al., BMC Immunol, 2017: 18: 39). These higher immune responses occur without detriment of the physiological parameters of the studied species while maintaining the autoregulated characteristics of the response. The antibody levels obtained while under the immunization schedules were significantly lower than those achieved after a bolus administration of therapeutic antibodies. Previously discussed elements point to a remaining potential for the improvement of response to the vaccine without inducing adverse effects.

Therefore is still an interest to obtain variants of human VEGF-A with increase anti-angiogenic, anti-tumoral, anti-metastatic, anti-inflammatory, or immune-restoring effects, that allows an improvement of the immune response achieves on individuals immunized with the antigenic preparations comprising such variants.

### Disclosure of the invention

This invention solved the previously mentioned problem by providing a polypeptide that comprises a functional mutant of a human VEGF-A isoform, folded in a non-natural re-arrangement of disulfide bridges, where the 2^{nd} and the 4^{th} cysteine are only forming intramolecular bonds, and the 7^{th} and 8^{th} cysteines of the same molecule are only found as part of intermolecular bridges.

For the invention, a functional mutant of VEGF-A is a molecule with a 95% sequence identity as compared to the natural variant, but differing from the former on its binding to VEGFR2, and the fact that it does not induce the signal transductions associated with the receptor binding.

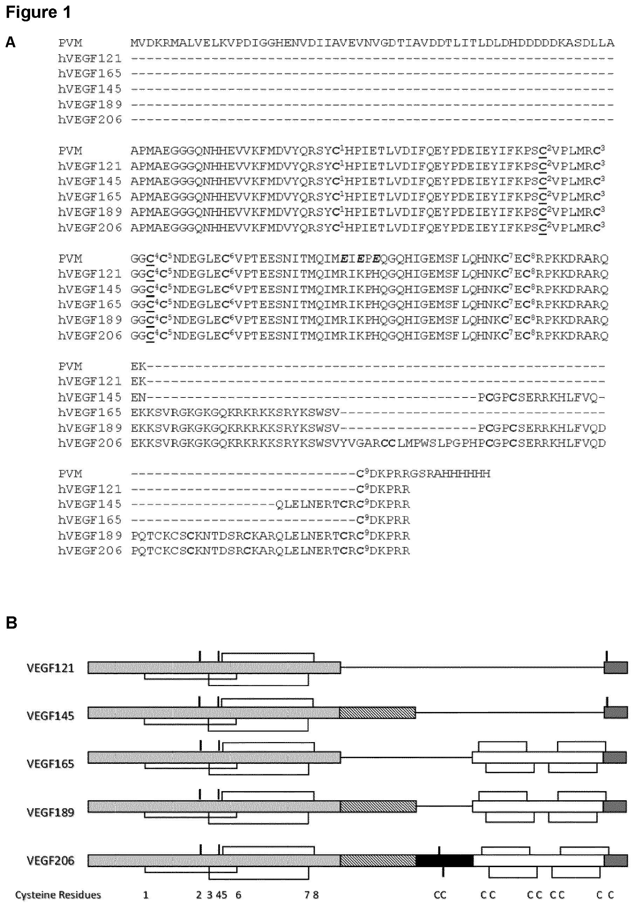

The polypeptides described herein are related to the proteins of human VEGF, in particular to VEGF-A and its isoforms, and has not been previously described. They were generated primarily, from the introduction of changes into the purification process of the polypeptide defined by SEQ ID NO:2 (Morera, et al., Angiogenesis, 2008: 11: 381-93). Their analyses indicated a gain in stability, immunogenicity, and antitumoral effects as compared to the original antigenic preparation PVM (Examples 1, 2, 3, 4 y 5). To obtain the invention related polypeptides comprising a functional mutated form of a human VEGF-A isoform, the amino acids involved in VEGFR2 binding were mutated. In one of the invention embodiments, the polypeptide is characterized by a human VEGF-A isoform selected from the group comprising VEGF-A¹²¹, VEGF-A¹⁴⁵, VEGF-A¹⁶⁵, VEGF-A^{189,} and VEGF-A²⁰⁶. The cysteine arrangement that increases the immunogenicity claimed in this invention, extends to those isoforms that share the canonic cysteine structures described for VEGF-A¹²¹ (Figure 1) and present the same therapeutic effects. Hence, the same cloning and expression systems were used to insert the sequences corresponding to VEGF-A¹⁴⁵, VEGF-A¹⁶⁵, VEGF-A^{189,} and VEGF-A²⁰⁶. Comparative evaluations between the products preserving the cysteine knot and those that do not, indicate that in absence of this canonic structure the increments on immunogenicity described for PVM and PVM-I can be replicated (Example 2).

In other embodiment, the polypeptide that comprises a functional mutant of a human VEGF isoform includes a re-arrangement of disulfide intramolecular bridges, form between the 1^{st} and the 2^{nd} cysteines, the 3^{rd} and the 4^{th} cysteines, and the 5^{th} and the 6^{th} cysteines; and intermolecular bonds form among cysteines 7^{th} of two polypeptide chains, cysteines 8^{th} of two polypeptide chains, and the last cysteine of two polypeptide chains.

In other embodiment, the polypeptide that comprises a functional mutant of a human VEGF isoform includes a re-arrangement of disulfide intramolecular bridges, form between the 1^{st} and the 2^{nd} cysteines, the 3^{rd} and the 4^{th} cysteines, and the 5^{th} and the 6^{th} cysteines; and intermolecular bonds form among cysteines 7^{th} and 8^{th} of two different polypeptide chains, and the last cysteine of two polypeptide chains.

In other embodiment, the polypeptide that comprises a functional mutant of a human VEGF isoform includes a re-arrangement of disulfide intramolecular bridges, form between the 1^{st} and the 2^{nd} cysteines, the 3^{rd} and the 5^{th} cysteines, and the 4^{th} and the 6^{th} cysteines; and intermolecular bonds form among cysteines 7^{th} of two polypeptide chains, cysteines 8^{th} of two polypeptide chains, and the last cysteine of two polypeptide chains.

In other embodiment, the polypeptide that comprises a functional mutant of a human VEGF isoform includes a re-arrangement of disulfide intramolecular bridges, form between the 1^{st} and the 2^{nd} cysteines, the 3^{rd} and the 5^{th} cysteines, and the 4^{th} and the 6^{th} cysteines; and intermolecular bonds form among cysteines 7^{th} and 8^{th} of two different polypeptide chains, and the last cysteine of two polypeptide chains.

In other embodiment, the polypeptide that comprises a functional mutant of a human VEGF isoform includes a re-arrangement of disulfide intramolecular bridges, form between the 1^{st} and the 2^{nd} cysteines, the 3^{rd} and the 6^{th} cysteines, and the 4^{th} and the 5^{th} cysteines; and intermolecular bonds form among cysteines 7^{th} of two polypeptide chains, cysteines 8^{th} of two polypeptide chains, and the last cysteine of two polypeptide chains.

In other embodiment, the polypeptide that comprises a functional mutant of a human VEGF isoform includes a re-arrangement of disulfide intramolecular bridges, form between the 1^{st} and the 2^{nd} cysteines, the 3^{rd} and the 6^{th} cysteines, and the 4^{th} and the 5^{th} cysteines; and intermolecular bonds form among cysteines 7^{th} and 8^{th} of two different polypeptide chains, and the last cysteine of two polypeptide chains.

In other embodiment, the polypeptide that comprises a functional mutant of a human VEGF isoform includes a re-arrangement of disulfide intramolecular bridges, form between the 1^{st} and the last cysteines, the 2^{nd} and the 3^{rd} cysteines, and the 4^{th} and the 5^{th} cysteines; and intermolecular bonds form among cysteines 7^{th} of two polypeptide chains, and between the cysteines 8^{th} of two polypeptide chains.

In other embodiment, the polypeptide that comprises a functional mutant of a human VEGF isoform includes a re-arrangement of disulfide intramolecular bridges, form between the 1^{st} and the last cysteines, the 2^{nd} and the 3^{rd} cysteines, and the 4^{th} and the 5^{th} cysteines; and intermolecular bonds form among cysteines 7^{th} and 8^{th} of two different polypeptide chains.

In other embodiment, the polypeptide that comprises a functional mutant of a human VEGF isoform includes a re-arrangement of disulfide intramolecular bridges, form between the 1^{st} and the last cysteines, the 2^{nd} and the 4^{th} cysteines, and the 3^{th} and the 5^{th} cysteines; and intermolecular bonds form among cysteines 7^{th} of two polypeptide chains, and between the cysteines 8^{th} of two polypeptide chains.

In other embodiment, the polypeptide that comprises a functional mutant of a human VEGF isoform includes a re-arrangement of disulfide intramolecular bridges, form between the 1^{st} and the last cysteines, the 2^{nd} and the 4^{th} cysteines, and the 3^{th} and the 5^{th} cysteines; and intermolecular bonds form among cysteines 7^{th} and 8^{th} of two different polypeptide chains.

In one embodiment, the polypeptide that comprises a functional mutant of a human VEGF isoform includes a re-arrangement of disulfide intramolecular bridges, form between the 1^{st} and the last cysteines, the 2^{nd} and the 5^{th} cysteines, and the 3^{th} and the 4^{th} cysteines; and intermolecular bonds form among cysteines 7^{th} of two polypeptide chains, and between the cysteines 8^{th} of two polypeptide chains.

In other embodiment, the polypeptide that comprises a functional mutant of a human VEGF isoform includes a re-arrangement of disulfide intramolecular bridges, form between the 1^{st} and the last cysteines, the 2^{nd} and the 5^{th} cysteines, and the 3^{th} and the 4^{th} cysteines; and intermolecular bonds form among cysteines 7^{th} and 8^{th} of two different polypeptide chains.

In a particular embodiment of the invention, the polypeptide that comprises a functional mutant of a human VEGF-A isoform comprise, additionally, an amino-terminal segment that increases its expression in bacteria and a carboxy-terminal fragment that facilitates the purification process.

Without restricting the scope of the invention and in a preferred embodiment, a recombinant protein isomer mixture which comprises 46 amino acids of the amino terminal fragment of the protein P64K from *Neisseria meningitidis* (SEQ ID NO: 24) are used. In said chimeric protein, the mentioned amino-terminal segment and the amino-terminal portion of VEGF isoform 121 are separated by thirteen amino acids that link these two segments. Furthermore, the protein has a carboxy-terminal six-histidine sequence useful for antigen production. The preservation within the fusion polypeptide of the Cys 110 and 119 which are equivalent to Cys 51 and 60 in natural VEGF-A (Figure 1), likely promotes the formation of several structural isomers with changes in the secondary structure of the protein. Only the previous works of Bequet-Romero *et al.*, and Morera *et al*., used the VEGF-A variant including these cysteines. Nevertheless, in any of them, intramolecular disulfide bridges were detected between cysteines 2 and 4 in the polypeptide chain. The presence of these two Cys and the process for antigen production where Cys 2 and 4 form only intramolecular bonds constitute a new approach to the generation of a protein with a vaccine purpose. Altogether the strategy leads to the exposition of sites evoking an increased immunologic response. The former might be due to the recognition by antibodies that neutralize VEGF-A binding to its type 2 receptors, or to the fact that the antigen digestion by the proteasome is facilitated, and hence new peptides can be presented by antigen-presenting cells.

Particular embodiments of this invention are the polypeptides with amino acid sequences including SEQ ID NO: 18 to SEQ ID NO: 23. They have increased immunogenic properties as compared to the polypeptide preserving the tertiary structure described for VEGF-A isoform 121. In those polypeptides modifications of the tertiary structure were detected that account for the desired superior effect. This superior immunogenicity was surprising since the changes in the secondary structure so far have been described as non-optimal for the generation of an effective immune response for human VEGF-A (Wentink, et al., Proc Natl Acad Sci USA, 2016: 113: 12532-7).

In a purification process established for the polypeptide identified by SEQ ID NO: 2 structural isomers of VEGF-A with Cys-knots structures, similar to those of the wild-type molecule are produced, as shown in Example 1. Unexpectedly, changes in the purification process lead to the enrichment of an isomer's family presenting intramolecular disulfide bonds involving Cys 110 and 119, corresponding to the Cys 51 and 60 from VEGF-A, that naturally form intermolecular bridges. In the antigenic preparation herein named PVM-I, other intermolecular bonds were observed involving Cys 161, 163, and 175, a fact related to the production of oligomeric structures stabilized by disulfide bridges. There's no information in the previous art about the formation, stabilization, and ordering of herein describe foldings. This is related to the fact that practically any study dealing with VEGF-A recombinant production preserved in the sequence the Cys residues equivalent to Cys 51 and 60 of the natural VEGF-A. When these cysteines were conserved in the primary sequence renaturalization processes were used to recreate the natural disulfide bridges (Pizarro, et al., Protein Expression and Purification, 2010: 72: 184-93). Therefore, the detection of new, non-canonical bonds, in the absence of the natural ones, and their relation with the increased stability and biological activity of PVM-I preparation, is an unexpected and surprising finding of this invention.

This invention is not restricted to a particular form for the production of the structural isomers present in the antigenic preparation PVM-I. As shown in example 7, these isomers can be obtained and separated using other procedures. Likewise, in this example, the equal contribution of the identified isomers is depicted in terms of immunogenicity increments. Several structural isoforms coexist within the protein fraction with lower resistance to trypsin-based digestion, and they display similar capabilities for the generation of an increased specific immune response.

PVM-I preparation is composed of isomers mixtures or by particular isomers isolated using reverse phase chromatography able to efficiently separate the 12 isomers variants. Any of the isomers mixes is potentially effective, since the administration of the separated isomers recreates to the immunological levels, the effect observed for the preparation that contains all of them (Example 7). This could be related to the fact that in all the cases the generation of peptides by endoproteases like trypsin is favored, and trypsin is one of the essential components of the proteasome system responsible for the generation of the peptides to be presented to the immune system in the frame of MHC molecules (Pamer, et al., Annu Rev Immunology 1998: 323-358).

Is also a subject of the present invention an antigenic preparation comprising at least a polypeptide of a functional mutant of a VEGF-A isoform folded in a non-natural re-arrangement of disulfide bridges, where the second and the fourth cysteines of the polypeptide chain are only forming intramolecular bonds, and the seventh and eighth cysteines of the polypeptide chain are only forming intermolecular bridges. The antigenic preparation comprises at least one pharmaceutically acceptable excipient or diluent. The immunogen dosage can be administered in vehicles accepted for pharmaceutical usage, that are not toxic and do not present intrinsic therapeutic effects. These vehicles include the ionic exchangers, alum, alum stearate, lecithin, serum proteins, buffer substances, glycine, sorbic acid, potassium sorbate, partial glyceride mixtures of fatty acids from vegetal origin, water, salts and electrolytes, polyvinylpyrrolidone, substance based on cellulose and polyethylene glycol. In the present invention, Tris Chloride buffer is preferentially used as a vehicle for the vaccine preparations.

The invention is not restricted to the antigenic preparations obtained by a given procedure since the main object of the invention is the polypeptides with a secondary/tertiary structure significantly different from those found in nature.

The isomers family composes of the polypeptides including mutants of VEGF-A isoforms can be obtained in a homogeneous composition using other strategies that include, among others, the purification of the structural isomers using reverse-phase high-pressure liquid chromatography (RP-HPLC) or the controlled renaturalization in the presence of a disbalance of denaturalization agents. As shown in Example 7, structural isoforms can be separated using RP-HPLC, and their independent use in identical concentrations results in a similar effect in terms of immunogenicity, antitumoral and antimetastatic effects as compared to the already described mixture (PVM-I).

PVM-I preparation is an antigenic variant with higher immunogenicity and stability according to the preliminary studies using Freund adjuvants (Example 1). These studies were extended to adjuvants and schemes of relevance for the therapeutic scenario in non-human mammals (Examples 2-5, y 9-13) and humans (Example 14 y 15). The use of an antigenic variant of higher stability is relevant to the productive and commercial context, while the increased immunogenicity offers a superior therapeutic solution (Examples 2-5, 9-15).

In a preferred embodiment, the invention also described a pharmaceutic composition that comprises a functional mutant of a human VEGF-A that folds in a non-natural rearrangement of disulfide bridges, where the second and the fourth cysteines of the polypeptide sequence are only forming intramolecular bonds while the seventh and the eight are only forming intermolecular bridges.

The compositions related to the invention contain at least a pharmaceutically accepted adjuvant. To increase the immune response, the structural isomers described in the invention can be combined with already described immunopotentiators. Among them are the mineral salts, immunostimulators like cytokines, molecular adjuvants (CD40, CD154, the invariant chain of MHC type I, LFA3), Saponins, Muramyl dipeptide derivatives, the oligonucleotides CpG, lipopolysaccharides, monophosphoryl lipid A and polyphosphase, lipidic particles (ie. Freund adjuvant, MF59, and Montanide), liposomes, nanoparticles, virosomes, ISCOMS, cochleates; microparticulate adjuvants, poloxamers, viral and bacterial antigens. Also, mucosal adjuvants are included. In a particular embodiment, the adjuvant is selected from the group composed of oil-based adjuvants, aluminum salts, proteoliposomes, and proteoliposomes conjugated to gangliosides. The administration of the antigen with pharmaceutically accepted adjuvants can contribute to avoiding the appearance of new metastases, and to the reduction and even the elimination of the primary tumor, as a first- or second-line treatment in combination or not with other antitumoral agents (Examples 2-5, 9-12, y 14). Also, this type of immunotherapy might be of use for the treatment of acute and chronic inflammatory processes (asthma, respiratory distress, endometriosis, atherosclerosis, tissue edema), infectious diseases (hepatitis, Kaposi's Sarcoma), autoimmune diseases (diabetes psoriasis, rheumatoid arthritis), diabetic retinopathy, macular degeneration, neovascular glaucomas, hemangiomas, and angiofibromas, among others (Examples 12, 13 and 15).

Particularly, the invention described the administration of vaccine antigens, in weekly schedules when formulated in a group of adjuvants under the name of NAcGM3-VSSP. These adjuvants can be obtained from the conjugation of the natural or synthetic forms of the ganglioside N-Acetyl GM3 to external membrane vesicles from *N. meningitidis* (Patent US6149921; International Application WO201986056A1; Regalado, et al., Organic Process Research & Development 2013: 17: 53-60). From herein, these adjuvant variants known also as VSSP (*Very Small Size Proteoliposomes*) are referred to as NAcGM3-VSSP. The ones incorporating the synthetic N-Acetyl GM3 differ among them in terms of the length of the fatty acid added to the ganglioside. In general, the use of the variants including stearic acid (sNAcGM3-VSSP) or oleic acid(oNAcGM3-VSSP) replicates the results obtained using the natural variant of the ganglioside in terms of Immunogenicity, antitumoral, and anti-metastatic effects. In all these conditions of formulation superior anti-tumoral, anti-metastatic, anti-inflammatory, and immune restoring effects were obtained when PVM-I was used as antigen as compared to PVM (Examples 4-12).

Histopathologic studies reveal in all the cases, a reduction in the number of functional blood vessels in the primary tumors, a decrease in the tumoral cells density, and a significant trend to the reduction of the mitotic/apoptosis ratio, that were inversely correlated to the tumoral growth. Considering that cancer-associated lethality is related mainly to metastatic events, the antigenic preparations were further evaluated in aggressive models of spontaneous and experimental metastases to the lungs. In all cases, PVM-I based preparation showed significantly better anti-metastatic profiles than those obtained with the vaccine antigen PVM.

The analyses of metastases foci in treated animals shown similar characteristics to those described for treated primary tumors, with a reduction in the blood vessel density and the mitotic/apoptotic balance, parallel to an increment in the number of necrotic foci that correlates with the number of metastases founded. Of interest, the vaccine-based treatment not only was able to reduce the number of metastases but also their size and proliferative capacity, indicating a dual effect of the therapeutic intervention on the metastatic process: the implantation of the foci and its growth (Example 9).

Of notice, the antigenic preparation from the invention is administered every two weeks when it is formulated in alum phosphate. With this adjuvant, superior anti-tumoral, anti-metastatic, anti-inflammatory, and immune restoring effects were obtained when PVM-I was used as antigen as compared to PVM.

At similar concentrations, and in all tested adjuvants PVM-I preparation is significantly superior to PVM, in terms of achieved specific antibody response and neutralizing capacity of the serum. These effects were observed in both mice strains used, indicating the wide spectrum response achievable with the therapy in the context of diverse haplotypes. Similarly, for NAcGM3-VSSP based formulation PVM-I is a superior antigen choice for the induction of cellular response, that directly eliminates syngeneic tumoral cells. The new antigenic preparation evidenced also higher immunogenicity when assayed in species sharing a higher homology for natural VEGF-A, like human primates.

For therapeutic applications, the vaccines from the present invention are administered to a mammal, preferably a human, in a pharmaceutically acceptable dosage using routes known for those skilled in the art. Described antigenic preparations can be administered in concomitant or sequential schedules with other treatments.

The administration of the new antigenic preparation PVM-I significantly reduce the growth of tumors of diverse origins, including melanoma, lung, breast, and colon carcinoma, These results were obtained in tumoral models that have been relevant for the translation of other antitumoral treatments to the clinical practice, indicating the applicability of this vaccine strategy for the clinical scenery in cancer treatment. The increments in antigen dosage are related to an increased biological effect associated with the immune response and hence the amount of antigen to be used for each application depends on the desired effect.

The invention also reveals for the first time the use of a polypeptide comprising a functional mutant of a human VEGF-A isoform that is folded in a non-natural re-arrangement of disulfide bridges, in the manufacture of a drug for the treatment of diseases related to the increment of angiogenesis, inflammation, and immunosuppression. In a particular embodiment, the disease to be treated is selected from a group comprising cancer, macular degeneration, diabetes, rheumatoid arthritis, and edema.

The new antigenic preparation PVM-I is useful for cancer active immunotherapy in humans. The use of the same as the treatment of neoplastic diseases reproduces the immunologic effects described in the pre-clinical phase. On immunized patients, VEGF-A specific antibodies were detected as well as clones of T lymphocytes that secrete IFN-gamma in response to VEGF-A stimulation. The presence of this immune response was associated with a significant increment in survival. Long-term immune responses were observed in the groups of patients with complete responses. This is an unusual finding for cancer vaccines. Furthermore, is interesting that the therapy success is not related to a complete plasmatic VEGF-A ablation. This element might be linked to the absence of side-effects similar to those described for other therapeutic alternatives targeting the VEGF/VEGFR2 system in both sceneries preclinical and clinical. Similarly, by achieving a reduction in circulating VEGF-A, and a systemic neutralization of the ability of VEGF-A to bind to VEGFR2, the new antigenic preparation, administered in a pharmaceutically acceptable adjuvant, can be used for the treatment of another group. of pathologies related to excessive angiogenesis.

The invention does not restrict the use of the antigenic preparations disclosed herein to particular diseases and illustrates how the therapy is effective in those contexts as well. Thus, the administration of the antigenic preparation of the invention in the presence of aluminum phosphate or sNAcGM3-VSSP rescue the immune system of tumor-bearing animals (Example 11). The antigenic preparation PVM-I is also shown to have an increased anti-inflammatory effect, as compared to the antigenic preparation referred to herein as PVM, in the context of a collagen-induced arthritis model in DBA / I mice (Example 12). Similarly, the antigenic preparation PVM-I had a greater anti-angiogenic effect in animal models of corneal damage, and in age-related macular degeneration in humans (Examples 13 and 15). This illustrates the potential utility of this strategy in the treatment of non-neoplastic diseases. The reduction in vascularization in the cornea, as a result of the administration of VEGF-A in this compartment, indicates the potential of the strategy for the treatment of age-related macular degeneration and diabetic retinopathy, diseases that present with an increase in local levels of VEGF-A, and which can be controlled from the immune response that induces the active immunization with preparations comprising the polypeptides of the invention.

In another aspect, the invention reveals the use of pharmaceutical compositions comprising an antigenic preparation with at least one polypeptide comprising a functional mutant of an isoform of human VEGF-A, which folds into an unnatural rearrangement of disulfide bridges, in restoring the immune system. The new antigenic preparation, in addition to presenting better immunogenic properties, results in a superior restoration of the immune system. An analysis of the suppressor myeloid and regulatory T cells was performed in the primary and metastatic lesions of the animals treated with the antigenic preparation of the invention. This analysis indicated that the number of these suppressor cells is reduced when the vaccine preparation is administered in different adjuvants and schedules. The functionality of the suppressor cells, as well as their number at the systemic level, is also affected (Example 10).

The adjuvant effect of the antigenic preparation disclosed in the invention was demonstrated in an experimental model expressing the Ovalbumin antigen (OVA). Administration, either sequential or combined, of the PVM-I vaccine antigen in an adjuvant with OVA, led to significant increases in the OVA-specific cellular and humoral immune response. Furthermore, the effects of vaccination with PVM-I on the cross-presentation of VEGF-A and OVA were observed, in the context of class I presenting molecules (Example 11).

### Figure Description

**Figure 1****. A:** Sequence alignment of the amino acid sequences from natural VEGF-A in its isoforms 121, 145, 165, 206 y 189, with the amino acid sequence present in the antigenic preparation PVM. Cysteines represented with a dark C **(C)** are those involved in intramolecular bridges, while the one further underlined **(C)** forms intermolecular bonds. **B:** Schematic representation of the disulfide bond arrangements in natural VEGF-A variants.

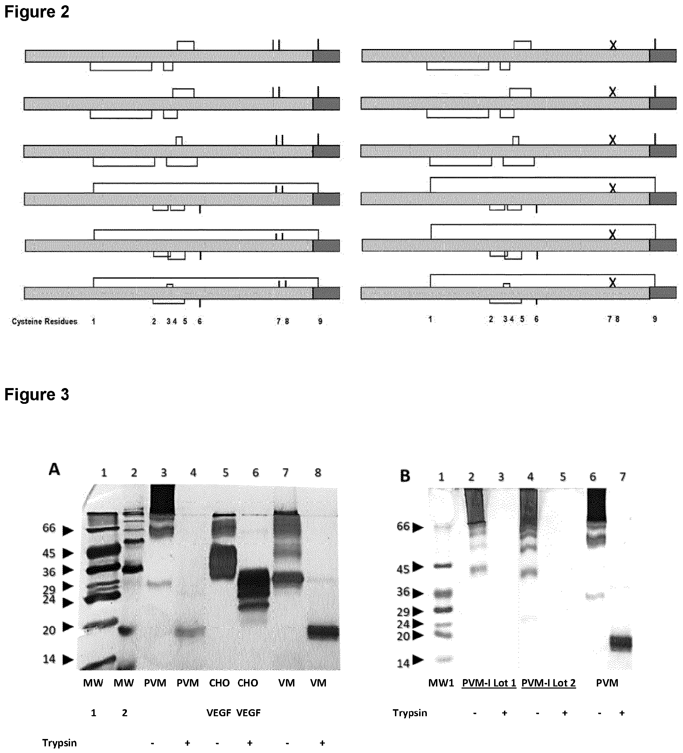

**Figure 2****.** Schematic representation of the disulfide bond detected in the antigenic preparation PVM-I.
**Figure 3****.** Evaluation of the presence of the canonic "cystine knot" from VEGF-A using native polyacrylamide gel electrophoresis (in absence of Dithiothreitol or Betamercaptoethanol). **A:** Analysis of PVM, CHO-VEGF-A, and VM proteins after trypsin digestion. **B:** Analysis of the proteins in two lots of PVM-I preparation (lanes 2-5) and PVM, as a control (lanes 6-7), after trypsin digestion. Molecular weight markers were run in lanes marked as PM1 and PM2.

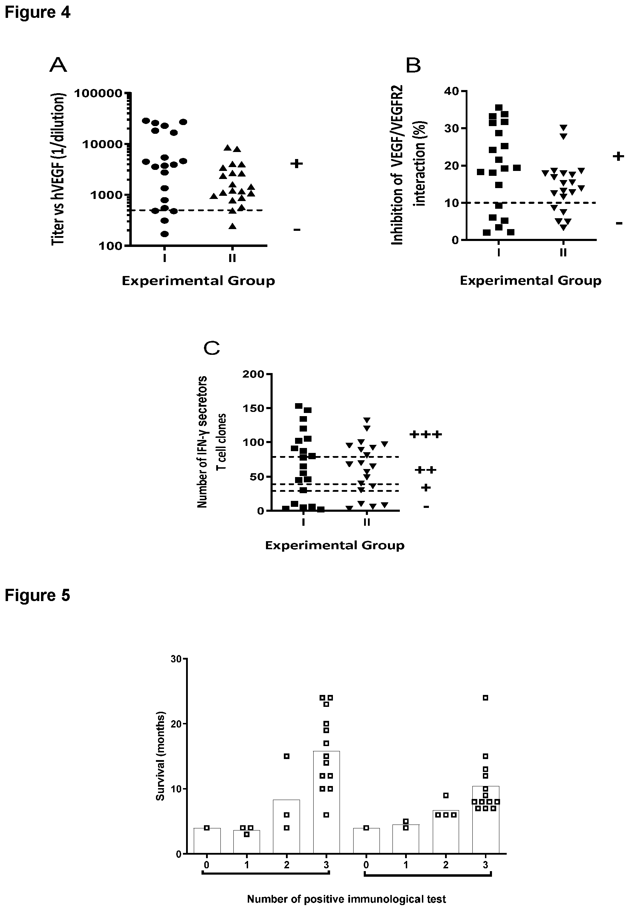

**Figure 4****.** Evaluation of the humoral and cellular response in advanced cancer patients immunized with PVM-I, administered in NAcGM3-VSSP adjuvant (I) or Alum Phosphate (II). **A:** VEGF-A specific IgG titer on immunized patients. **B:** Study of the ability of immunized patient serum to block VEGF-VEGFR2 interaction. **C:** Evaluation of the VEGF specific cellular response, measured by INF-gamma ELISPOT. In all the cases values represent the differences found as compared to values at treatment start.
**Figure 5****.** Analyses of the immune response and its impact on the survival of patients immunized with the antigenic preparation PVM-I. Survival time is shown as a function of the positive immune responses detected for the group receiving the antigen either on NAcGM3-VSSP adjuvant (I) or in Alum Phosphate (II).

### EXAMPLES

### Example 1. Purification and characterization of PVM and PVM-I antigenic preparations.

The DNA encoding isoform 121 of human VEGF-A, where amino acids 82, 84 and 86 were mutated by glutamic acid, was cloned in the vector PM238 (Morera, et al., Angiogenesis, 2008: 11: 381-93). A hundred percent of the DNA sequence was verified and designated as SEQ ID NO: 1. In this genetic construction, the gene for resistance to ampicillin was interrupted by the one corresponding to resistance to kanamycin, without changes in expression levels. The plasmid was transformed into the Escherichia coli strain BL21, and the transformants with the highest expression levels were selected in a chemically defined medium. This medium was designed to maximize the expression of the recombinant protein, in the absence of components of animal origin. This protein was purified according to that described by Morera et al., (Morera, et al., Angiogenesis, 2008: 11: 381-93). Briefly, the protein was extracted in 50 mM NaH2PO4 buffer; 300 mM NaCl; 6M urea; pH 7.8; for 16 hours at 4°C, and purified by nickel affinity chromatography, according to the manufacturer's instructions (QIAGEN). The buffer was changed to 10 mM Tris at pH 7.4 in size exclusion chromatography on matrix G25 (GE Healthcare).

The resulting protein preparation was evaluated by mass spectrometry to verify its amino acid sequence. The ESI-MS and ESI-MS / MS (Electrospray ionization mass spectrometry and Electrospray ionization tandem mass spectrometry, respectively), were obtained in a QTOF-2 orthogonal hybrid configuration spectrometer (Micromass, United Kingdom), with Z-spray electrospray ionization source (nanoESI). The molecular mass of the reduced protein preparation (21 569.13 Da) corresponded to the theoretical estimate (21569.31 Da) for the isoform 121 of human VEGF-A, fused to a fragment of the bacterial protein P64K at the amino-terminal end and separated from it by 13 amino acids that function as a bridge between these polypeptides, also incorporating a histidine tail at the carboxyl-terminal end. The mutations introduced to the human VEGF-A sequence were checked and glutamic acid was detected instead of the amino acids corresponding to the natural VEGF-A sequence at positions 141, 143, and 145: Arginine, Lysine, and Histidine respectively.

The integrity of the amino-terminal end in the sample was verified from the mixture of peptides obtained from the digestion with Glu-C endoproteinase. In the ESI-MS spectrum, the signal corresponding to the peptide ¹VDKRMALVE⁹ (double charged, theoretical m/z 530.78) was observed. This peptide was sequenced by ESI-MS/MS, and the sequence was identical to that expected for the amino-terminal end of the protein.

In the ESI-MS spectrum of the reduced intact protein, a signal corresponding to the C-terminal end was detected, by increasing the voltage at the input cone of the mass spectrometer, causing fragmentation at the source. Analysis of the result was consistent with the peptide ¹⁷⁷KPRRGSRAHHHHHH¹⁹⁰ (double charged, m/z 875.46) after sequencing by ESI-MS/MS, confirming the carboxyl-terminal sequence. Overall, the results of the sequencing allowed us to verify the primary structure of the polypeptide present in the antigenic preparation (SEQ ID NO: 2). Hereinafter the protein preparation defined by SEQ ID NO: 2 is called PVM.

A study of the fermentation and purification conditions indicated a recovery superior to the previously described process (Morera, et al., Angiogenesis, 2008: 11: 381-93), by modifying the growth conditions during fermentation, shortening the time of extraction, adjusting the specific protein loading in nickel affinity chromatography, and incorporating detergents into the washing steps. Briefly, during the fermentation process, the fermentation temperature of the growth phase was modified to 28°C, and the expression was induced only by increasing temperature to 37°C, without the need of chemical inducers. The protein extraction time from biomass in 6M Urea was also reduced from 16 to 2 hours, and a wash step was introduced in the nickel affinity chromatography with 0.1% Triton X114. Additionally, the preparation resulting from the molecular exclusion chromatography was formulated in mannitol (40 mg/mL), sucrose (10 mg/mL), and 10 mM Tris-HCl pH 7.4. This final protein preparation was called PVM-I, and hereinafter it is compared with the preparation obtained in the initial process, the PVM preparation.

In both preparations, the concentration of total proteins was evaluated, using the micro-coomassie method at a double wavelength (620 and 450 nm); and percent purity on SDS polyacrylamide gel electrophoresis (SDS-PAGE). Immuno-identification was performed by immunoblotting with monoclonal antibodies that recognize the different segments present in the antigen. In these studies the PVM and PVM-I preparations were identical.

The analysis of the conformation and stability of these lyophilized protein preparations were performed using molecular exclusion chromatography on an analytical scale, on a Superdex 200 XK 10/300 column (GE-Healthcare). The buffer Tris-HCl 10 mM, NaCl 150 mM, pH 7.4, was used as the mobile phase; at a flow rate of 0.5 mL/min. The lyophilized protein preparations were suspended in one milliliter of water, and the comparison data indicated that PVM and PVM-I present the same retention profile in this type of chromatography, at the time of reconstitution. In both cases, soluble aggregates with a molecular weight greater than 670 kDa are formed, according to the molecular weight standards used. However, the reconstituted solution from PVM-I showed differential characteristics, in terms of stability over time of the added composition, indicative of variations in its structural composition. The PVM-I preparation, after being reconstituted, maintains its retention time profile, in the molecular exclusion chromatography, for 30 days at 4°C, while the PVM preparation begins to lose stability, significantly, at 72 hours (Table 1). The conformational change in more than 14% of the PVM protein mass, at 72 hours, increases significantly at 30 days, when the original conformation is lost in more than 45%, according to an analysis of the area under the curve in the chromatographic charts.

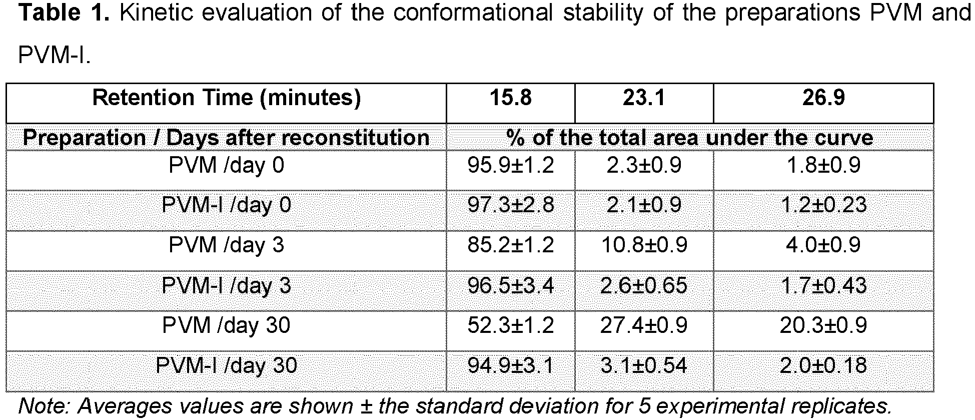

To evaluate the immunogenicity of the PVM and PVM-I protein preparations the humoral immune response produced in mice was assessed. Ten animals were used per group, from two mouse strains (C57BI/6 and BALB/c), and 100 µg of the protein preparations were administered, in 250 µL of total volume (protein/adjuvant ratio 1:1 v/v) on complete Freund's adjuvant (SIGMA) in the first dose, and on incomplete Freund's adjuvant (SIGMA) in the second dose, with a week interval. The serum from the animals was collected one week after the second immunization. The specific antibody titers for human VEGF-A present in the sera, and their ability to neutralize the interaction of VEGF-A with its type 2 receptor, were evaluated by ELISA, as previously described (Bequet-Romero, et al., Vaccine, 2012: 30: 1790-9). Titer results are expressed as the highest dilution at which the presence of specific antibodies for VEGF-A is detected. The neutralizing capacity is expressed as the percentage of the maximum VEGF-A / VEGFR2 binding that the antibodies present in the serum can eliminate.

When comparing immunogenicity, a significant increase in the seroconversion levels achieved when immunizing with the new antigenic preparation (PVM-I) was evidenced, compared to the previous one (PVM), even when equal amounts of these were administered, as shown in Table 2.

**Table 2. Immunogenicity of preparations PVM and PVM-I.**

| | **VEGF-A specific IgG Titers (1/Dilution)** | | |
|---|---|---|---|
| | **PVM** | **PVM-I** | **t-Student** |
| **C57BI/6** | 10655 ± 776 | 18655 ± 334 | < *0,0001* |
| **BALB/c** | 21399 ± 2343 | 26648 ± 2749 | *0,0002* |

| | **Percentage inhibition of VEGF-A binding to VEGFR2** | | |
|---|---|---|---|
| | **PVM** | **PVM-I** | **t-Student** |
| **C57BI/6** | 36,7 ± 3,5 | 51,6 ± 2,3 | < *0,0001* |
| **BALB/c** | 55,9 ± 7,7 | 67,8 ± 8,4 | *0,004* |

| | | | |
|---|---|---|---|
| *Note: IgG titers are expressed 1: Dilution. Average values are shown* ± *Standard Deviations from the mean and the p values correspond to the Student t-test for each mice strain.* | | | |

The formation of disulfide bridges was studied in both preparations taking into consideration that **(a)** the monomer of these proteic preparations have identical primary sequences (SEQ ID NO:2), **(b)** they present with 9 cysteine residues, and **(c)** that the immunogenicity, thermal and conformational stability of the human VEGF-A (which constitute 63% of PVM and PVM-I polypeptide sequences) is associated to the formation of a canonic cysteine structure, extensively characterized for the wild type protein and its family.

The ordering of the disulfide bridges within the protein preparations was preliminarily evaluated, through a study of proteolysis with trypsin, and it was analyzed by electrophoresis under non-denaturing conditions, and by molecular exclusion chromatography, on a Superose 12 XK 10/300 column, with 200 mM Tris-HCl, pH 8.0 as a mobile phase, at 0.5 mL/min.

It is known that the characteristic arrangement of cysteines in natural VEGF-A makes it resistant to tryptic digestions (Keck, et al., Arch Biochem Biophys, 1997: 344: 103-13), hence we conduct a comparison of the digestion of PVM and PVM-I with this enzyme. To this end, the final purification buffer was exchanged to 200 mM Tris-HCl pH 8.0 and the proteins were incubated at 37°C in the presence or absence of trypsin, at a 50:1 ratio (protein: trypsin) for 16 hours. Later, samples were taken to evaluate the efficiency of tryptic digestion by non-denaturing native electrophoresis (Figure 3). The following samples were used as controls for the correct conformation of the bridges in the VEGF-A molecule: 1) VM protein: the isoform 121 of human VEGF-A with the R80, K82, and H84 mutations to E produced from the periplasm of E. coli (Gavilondo, et al., Vaccine, 2014: 32: 2241-50), and 2) CHO-VEGF protein: isoform 121 of human VEGF-A obtained from the transfection of the eukaryotic cell line CHO (Chinese Hamster Ovary), (Sánchez Ramirez, et al., J Immunoassay Immunochem, 2016: 37: 636-58). These VEGF-A variants were purified by non-denaturing processes in the absence of a reducing agent. Figure 3 shows how the preparation from the bacterial periplasm is resistant to digestion with trypsin since 90% of it is recovered in a fraction that migrates with a molecular weight lower than that of the undigested protein, that agreed with the estimated molecular weight and the presence of the canonical cysteine structure, also known as a "cysteine knot", in the dimeric form of the mutant with or without the P64K segment (19.902 kDa) (Figure 3A).

The analysis of the PVM preparation showed a similar phenomenon, which is not observed for the PVM-I preparation, where the peptide corresponding to the cysteine knot does not appear (Figure 3B). Considering this, the peptides resulting from the digestion of the PVM-I preparation with trypsin or GluC were analyzed by mass spectrometry, as described for PVM, but without subjecting the sample to reduction processes. Table 3 depicts the disulfide bonds detected.

**Table 3. Summary of the monoisotopic molecular mass of the peptides generated during PVM-I digestion.**

| **m/z Theoric** | **z** | **m/z Experimental** | **Assignment** |
|---|---|---|---|
| 1272.93 | 3 | 1272.92 | ⁸³S-M¹¹⁴ peptide with intramolecular S-S between cysteines 85 and 110. |
| 993.98 | 4 | 993.96 | ⁸³S-R¹¹⁵ peptide with intramolecular S-S between cysteines 85 and 110. |
| 1324.97 | 3 | 1324.95 | |
| 1251.78 | 4 | 1251.74 | ¹¹⁶C-K¹⁶⁰ peptide containing the mutations R₁₄₁→E, K₁₄₃→E y H₁₄₅→E, and two intramolecular S-S between cysteines 116, 119, 120, and 127. |
| 927.44 | 4 | 927.44 | (⁸³S-K¹⁰⁷)-S-S-(¹⁷⁵C-R¹⁷⁹) peptide from the tryptic digestion containing a disulfide intramolecular bond between cysteines 85 and 175. |
| 1236 | 3 | 1236 | |
| 733.31 | 2 | 733.35 | (¹⁶¹C-K¹⁶⁶)-S-S-(¹⁶¹C-K¹⁶⁶) peptide from the tryptic digestion containing two intermolecular disulfide bonds between cysteines 161, 163, 161, and 163. |
| 617.30 | 2 | 617.31 | (¹⁷⁵C-R¹⁷⁹)-S-S-(¹⁷⁵C-R¹⁷⁹) peptide from the tryptic digestion containing an intermolecular disulfide bond between cysteines 175 and 175 of another molecule. |
| 1036.70 | 5 | 1036.74 | (⁷⁹V-E¹⁰³)-S-S-(¹⁷⁴K-H¹⁹⁰) peptide from a Glu-C based digestion, containing a disulfide intramolecular bond between cysteines 85 and 175. |
| 864.1 | 6 | 864.12 | |
| 744.32 | 3 | 744.31 | ¹⁰⁴Y-E¹²³ peptide from a Glu-C based digestion, containing two disulfide intramolecular bonds between cysteines 110, 116, 119, and 120. |
| 824.03 | 3 | 824.03 | (¹⁵³M-E¹⁶²)-S-S-(¹⁵³M-E¹⁶²) peptide from a Glu-C based digestion, containing a disulfide intermolecular bond between cysteines 161 and 161 of another molecule. |

The consistency of the existence of the mentioned disulfide bonds was analyzed and verified in a total of 8 lots of final product. The structures identified appears in both, the active pharmaceutical ingredient and the final formulation. The particular arrays of disulfide bridges found for PVM-I, significantly differ from the ones described for the wild type molecules of VEGF-A 121 and other isoforms of this protein. They are also different from the arrays described for the proteins when they are obtained by recombinant techniques in diverse prokaryotic and eukaryotic expression systems (Keck, et al., Arch Biochem Biophys, 1997: 344: 103-13). An exhaustive search was performed for intermolecular bridges between cysteines 110 and 119, corresponding to cysteines second and fourth of the natural VEGF-A. Even when in the wild-type molecule these cysteine residues form intramolecular bonds, the peptides corresponding to them were not detected on trypsin or Glu-C digested PVM-I preparation in any of the experimental conditions.

According to previous art, the most stable structures for VEGF-A correspond to those preserving the so call "cysteine knot". However, the studies conducted with the protein preparations PVM and PVM-I showed that in the conditions they are purified, the higher stability is associated with variants lacking the natural structures. Likewise, and despite the data from Timmerman et al., (Patent Application US2012/0231000), the comparative analysis of PVM-I immunogenicity in Freund adjuvant as compare to PVM indicates that the former, comprising the isomers mixtures from figure 2, is more immunogenic.

This study indicates that, in PVM-I preparation as compare to PVM, a change in disulfide bond disposition occurs, resulting in higher stability and reproducibility according to the new protocol for antigen production. Figure 2 shows the twelve variants of bridges established in a stable form among the nine cysteines detected in the polypeptide sequence of PVM-I.

### Example 2. Comparison of the immunogenicity of the antigenic preparations PVM and PVM-I administered in NAcGM3-VSSP.

Mice strains C56BL/6 and BALB/c differing in their abilities to generate a humoral or cellular immune response to an antigenic challenge were used. In both cases, 100 µg of antigenic preparation was administered per dose, in a) 100 µg of NAcGM3-VSSP incorporating natural ganglioside, b) 100 µg of sNAcGM3-VSSP incorporating ganglioside with stearic acid, or, c) 100 µg oNAcGM3-VSSP incorporating the ganglioside with oleic acid. 10 animals per group were immunized for 8 weeks, on a weekly schedule. The serum of the animals was collected one week after each immunization, after the third, and until the eighth. The antibody titers specific for human VEGF-A and their ability to neutralize the interaction of VEGF-A with its type 2 receptor were evaluated by ELISA, as described (Bequet-Romero, et al., Vaccine, 2012: 30: 1790-9).

Specific IgG titers increased with the increment in the number of immunizations, reaching a maximum at one week after the eighth immunization. The results corresponding to this experimental point are shown in Table 4. As can be seen, in both strains, after the eighth immunization, significantly superior results are obtained for the PVM-I antigenic preparation, both in terms of specific titers and inhibition of the binding of VEGF-A to its receptor. Comparative analysis of the three variants of the adjuvant NAcGM3-VSSP indicates that the vaccine preparations are equally immunogenic.

**Table 4. Results of the immunogenicity evaluation for PVM and PVM-I antigenic preparations.**

| | **VEGF-A specific IgG titer (1:Dilution)** | | |
|---|---|---|---|
| NAc-GM3 VSSP | **PVM** | **PVM-I** | **t-Student** |
| **C57BI/6** | 6500 ± 221 | 8592 ± 631 | *< 0.0001* |
| **BALB/c** | 29785 ± 3662 | *38870* ± 5574 | *< 0.0001* |
| sNAc-GM3 VSSP | | | |
| **C57BI/6** | 5900 ± 634 | 7664 ± 547 | *< 0.0001* |
| **BALB/c** | 25879 ± 2669 | *35990* ± 2899 | *< 0.0001* |
| oNAc-GM3 VSSP | | | |
| **C57BI/6** | 6205 ± 554 | *8120* ± *421* | *< 0.0001* |
| **BALB/c** | 30879 ± 4100 | *37555* ± *3552* | *< 0.0001* |

| | **Percentage of inhibition of VEGF-A binding to VEGFR2** | | |
|---|---|---|---|
| NAc-GM3 VSSP | **PVM** | PVM-I | t-Student |
| **C57BI/6** | 25.8 ± 4.5 | 39.9 ± 2.2 | *< 0.0001* |
| **BALB/c** | 62.2 ± 5.9 | 79.4 ± 3.1 | *< 0.0001* |
| sNAc-GM3 VSSP | | | |
| **C57BI/6** | 21.4 ± 2.3 | 37.3 ± 3.5 | *< 0.0001* |
| **BALB/c** | 60.2 ± 1.9 | 77.3 ± 3.6 | *< 0.0001* |
| oNAc-GM3 VSSP | | | |
| **C57BI/6** | 27.5 ± 1.9 | 38.5 ± 3.3 | *< 0.0001* |
| **BALB/c** | 60.2 ± 1.9 | 77.3 ± 3.5 | *< 0.0001* |

| | | | |
|---|---|---|---|
| *Note: the p-value corresponding to the Student t-test is shown.* | | | |

### Example 3. Comparison of the immunogenicity of the antigenic preparations PVM and PVM-I administered in alum phosphate.

Mouse strains C56BL/6 and BALB/c were used. A hundred micrograms of the antigenic preparation were administered per dose; in 0.7 mg equivalent of Al3+ (aluminum phosphate). Ten animals were immunized per group, with a bi-weekly frequency, for a total of 4 immunizations. The serum of the animals was collected a week after the second and fourth immunization. The human VEGF-A specific antibody titers, and their ability to neutralize the interaction of VEGF-A with VEGFR2, were evaluated by ELISA, as described (Bequet-Romero, et al., Vaccine, 2012: 30: 1790-9).

An increase in titers was observed as the number of doses administered was incremented for both antigen types and in both mouse strains. The results for the serum sample obtained after the fourth immunization are shown in Table 5. For both strains, after the fourth immunization, significantly superior results are obtained for the PVM-I antigenic preparation, in terms of specific antibody titers and of inhibition of VEGF-A binding to its receptor.

**Table 5. Analyses of the immunogenicity results for PVM and PVM-I.**

| | **VEGF-A specific IgG titer (1:Dilution)** | | |
|---|---|---|---|
| | **PVM** | **PVM-I** | **Student-t** |
| **C57BI/6** | 15560 ± 781 | 29576 ± 891 | 0.001 |
| **BALB/c** | 53000 ± 1799 | 70660 ± 3469 | < 0.0001 |

| | **Percentage of inhibition of VEGF-A binding to VEGFR2** | | |
|---|---|---|---|
| | **PVM** | **PVM-I** | **Student-t** |
| **C57BI/6** | 39.7 ± 3.8 | 55.3 ± 5.6 | 0.001 |
| **BALB/c** | 65.3 ± 6.5 | 76.5 ± 4.9 | 0.006 |

| | | | |
|---|---|---|---|
| *Note: the p-value corresponding to the Student t-test is shown.* | | | |

### Example 4. Evaluation of the cellular response induced by the immunization with the antigenic preparations of PVM or PVM-I administered in NAcGM3-VSSP.

The cellular response was analyzed as described by Bequet-Romero et al., (Bequet-Romero, et al., Angiogenesis, 2007: 10: 23-34; Morera, et al., Vaccine, 2012: 30: 368-77). In this case, the response was only assessed in the context of adjuvant NAcGM3-VSSP. The animals (n = 10, per group) were immunized in the scheme described for this adjuvant in Example 2, they were sacrificed one week after the last immunization, and the cells isolated from mice spleens were co-incubated with syngeneic tumor cells, labeled with the CFSE (carboxyfluorescein succinimidyl ester) fluorophore, in a 100:1 ratio (effector cells: labeled tumor cells).

Melanoma cell line B16F10, EL4 lymphoma, and lung carcinoma 3LL-D122 syngeneic with the strain C56BL/6 and the colon carcinoma lines CT26, breast F3II and renal RENCA syngeneic with the BALB/c strain were chosen for this study. The analysis by flow cytometry (Space ML-PARTEC) of the viable cells after co-incubation showed that, in both mouse strains, the administration of the PVM-I preparation resulted in direct cellular response of greater strength, even when similar doses of the two antigenic preparations were administered. Table 6 shows the results corresponding to the percentage of tumor cells that die when confronted with the lymphocytes of the immunized animals, taking as 100% viability the average value of cells corresponding to treatment with excipients. In both mouse strains (C57BI/6 and BALB/c the administration of the antigenic preparation PVM-I (in all cases corresponding to group IV) is superior to the use of the PVM preparation, in terms of induction of an effective cellular response.

**Table 6. Evaluation of cell-mediated cytotoxicity in response to PVM and PVM-I administration in NAcGM3-VSSP for mice strains BALB/c and C57BI/6.**

| **Treatment Group** | **(I) No Treatment** | **(II) NAcGM3-VSSP PVM** | **(III) NAcGM3-VSSP PVM-I** | **(IV) NAcGM3-VSSP Excipients** |
|---|---|---|---|---|
| **Strain C57BI/6** | | | | |
| **B16F10** | 1.498 ± 6.080 *^{a}* | 27.14 ± 3.034 *^{b}* | 45.52 ± 11.92 *^{c}* | 0.8686 ± 6.24 *^{a}* |
| **EL-4** | 8.440 ± 2.532 *^{a}* | 37.38 ± 1.909 *^{b}* | 49.65 ± 5.658 *^{c}* | 10.05 ± 6.560 *^{a}* |
| **3LL-D122** | 5.978 ± 7.115 *^{a}* | 23.80 ± 7.287 *^{b}* | 41.34 ± 2.160 *^{c}* | 0.1457 ± 6.552 *^{a}* |

| **Strain BALB/c** | | | | |
|---|---|---|---|---|
| **CT26** | 4.891 ± 6.341 *^{a}* | 24.03 ±7.184 *^{b}* | 47.33 ± 6.384 *^{c}* | 8.482 ± 4.804 *^{a}* |
| **F3II** | 9.588 ± 6.578 *^{a}* | 40.97 ± 9.524 *^{b}* | 69.65 ± 3.678 *^{c}* | 11.97 ± 5.824 *^{a}* |
| **RENCA** | 1.051 ± 2.996 *^{a}* | 34.73 ± 10.52 *^{b}* | 55.96 ± 7.523 *^{c}* | 0.8838 ± 3.230 *^{a}* |

| | | | | |
|---|---|---|---|---|
| *Note: Statistical comparison using Dunnet post-test: different letters indicate significant differences (p<0,05), in increasing order according to the value of the mean.* | | | | |

### Example 5. Efficacy of PVM and PVM-I based immunization for the treatment of solid tumors in mice.

In all cases, groups of 12 mice were immunized. The results of immunization with sNAcGM3-VSSP or with aluminum phosphate are described for the two tumor models. In the case where adjuvant sNAcGM3-VSSP was used, the animals were immunized subcutaneously, for 8 weeks, at a weekly dose of 200 µL total, containing 100 µg of the corresponding antigen and 100 µg of the adjuvant. For aluminum phosphate, the animals were immunized 4 times, on a bi-weekly schedule. Each time 100 µg of antigen was administered in a total volume of 200 µl, containing 0.7 mg equivalent of Al3 +, in the form of aluminum phosphate.

In the mouse strain C57BL/6, the melanoma model B16F10 was evaluated. Three days after the fourth immunization (in the presence of sNAcGM3-VSSP), or the second immunization (in the presence of aluminum phosphate), the mice were inoculated, subcutaneously, with a total of 20000 cells, in 100 µL of DMEM culture medium. Table 7 shows the weight of the primary tumor removed by surgery from the euthanized animals, 25 days after the tumor challenge.

**Table 7. Comparative evaluation of the administration of antigenic preparations PVM and PVM-I in sNAcGM3-VSSP and aluminum phosphate in the subcutaneous melanoma model B16F10.**

| **Treatment** | **Tumor Weight (g)** | **Inhibition of VEGF-A/VEGFR2 binding** | **Cellular response (% lysis)** |
|---|---|---|---|
| No Treatment | 2.523 ± 0.3705 *^{a}* | 2.4 ± 2.1 ^{c} | 5 ± 2.3 ^{c} |
| sNAcGM3-VSSP PVM | 1.748 ± 0.6057 *^{b}* | 47.3 ± 3.5 ^{b} | 40 ± 7.1 ^{b} |
| sNAcGM3-VSSP PVM-1 | 1.199 ± 0.3434 *^{c}* | 75.8 ± 4.5 ^{a} | 62 ± 8.4 ^{a} |
| sNAcGM3-VSSP Excipients | 2.933 ± 0.2133 *^{a}* | 4.1 ± 2.1 ^{c} | 12 ± 3.8 ^{c} |
| Alum Phosphate PVM | 1.822 ± 0.5987 *^{b}* | 59.2 ± 1.9 ^{b} | 35 ± 6.5 ^{b} |
| Alum Phosphate PVM-1 | 1.266 ± 0.6532 *^{c}* | 80.2 ± 1.9 ^{a} | 55 ± 5.4 ^{a} |
| Alum Phosphate Excipients | 2.941 ± 0.5778 *^{a}* | 2.2 ± 1.3 ^{c} | 8 ± 3.8 ^{c} |

| | | | |
|---|---|---|---|
| *Note: Statistical comparison using Dunnet post-test: different letters indicate significant differences(p<0,05), in increasing order according to the value of the mean.* | | | |

As observed in Table 7, the treatment of the animals with the antigenic preparations, in both adjuvants, significantly reduces tumor growth, towards day 25 after the tumor challenge, but it is the use of the new antigenic preparation ( PVM-I) which shows a more significant inhibition. This anti-tumor response was correlated with the presence of a specific immune response to VEGF-A, both humoral and cellular (p<0.05; Pearson's test).

Similarly, the anti-tumor effect was evaluated in the mouse strain BALB/c, using the same immunization scheme. The tumor challenge also occurred three days after the fourth immunization (in the presence of NAcGM3-VSSP) or the second immunization (in the presence of aluminum phosphate) with 25000 cells of the CT26 syngeneic colon carcinoma with this strain. Comparative analysis of tumor growth, 30 days after challenge, indicates a superior anti-tumor effect due to immunization with the antigenic preparation PVM-I, compared to the use of the PVM preparation (Table 8).

**Table 8. Comparative evaluation of the administration of the antigenic preparations PVM and PVM-I in NAcGM3-VSSP and aluminum phosphate in the colon carcinoma model CT26.**

| **Treatment** | **Tumor Weight (g)** | **Inhibition of VEGF-A/VEGFR2 binding** | **Cellular response (% lysis)** |
|---|---|---|---|
| No Treatment | 3.146 ± 0.3692 *^{a}* | 5.4 ± 2.3 ^{c} | 4.3 ± 1.8 ^{c} |
| PVM in sNAcGM3-VSSP | 2.463 ± 0.6314 *^{b}* | **39.3 ± 2.5 ^{b}** | **35 ± 6.3 ^{b}** |
| PVM-I in sNAcGM3-VSSP | 1.889 ± 0.4538 *^{c}* | **65.1 ± 11.5 ^{a}** | **59 5.5 ^{a}** |
| Excipients in sNAcGM3-VSSP | 3.503 ± 0.6455 *^{a}* | 8.3 ± 2.8 ^{c} | 5 ± 1.8 ^{c} |
| PVM in alum phosphate | 2.315 ± 0.7604 *^{b}* | **49.2 ± 8.9 ^{b}** | **28 ± 4.5** ^{b} |
| PVM-I in alum phosphate | 1.523 ± 0.5793 *^{c}* | **76.2 ± 10.9 ^{a}** | **50 ± 5.4 ^{a}** |
| Excipients in alum phosphate | 3.614 ± 0.5455 *^{a}* | 6.2 ± 4.3 ^{c} | 7 ± 2.2 ^{c} |

| | | | |
|---|---|---|---|
| *Note: Statistical comparison using Dunnet post-test: different letters indicate significant differences(p<0,05), in increasing order according to the value of the mean.* | | | |

Similar to the previous study, this result was correlated with the presence of both a humoral and cellular immune response specific to VEGF-A, superior in the case of the use of the antigenic preparation PVM-I (p<0.05; test Pearson). In both studies, the histological analysis of serial sections of the extracted tumors showed an anti-angiogenic, pro-apoptotic, and anti-proliferative effect of vaccination that was significantly more favorable to the use of the antigenic preparation PVM-I (p< 0.05, Dunnet in all analyzes).

### Example 6. Preparation and characterization of the polypeptide variants based on the isoforms 145, 165, 189, and 206 from VEGF-A.

As the alignment in Figure 1 shows, all the isoforms of VEGF-A share the cysteine residues present in isoform 121, so the strategy used to increase immunogenicity might be valid for the whole protein family. To validate this hypothesis, the isoforms 145, 165, 189, and 206 were cloned from the mRNA (messenger Ribonucleic Acid) of HeLa tumor cells. The automatic sequencing verified the sequences, according to those reported in the CCDS database (https://www.ncbi.nlm.nih.gov/CCDS/CcdsBrowse.cgi Release 22), according to its June 2018 update (see 12/23/2019).

Briefly, the strategy described in Example 1 was used, since all the isoforms of VEGF-A can be obtained in a single amplification reaction, and they are perfectly separable, due to their different sizes. In all cases, the mutation described in Example 1 was introduced, to rule out induction of VEGFR2 activation, from the administration of the antigenic preparation. The amino acid sequences corresponding to the proteins that generated the protein variants PVM145, PVM165, PVM189, PVM206 were named SEQ ID NO: 3, SEQ ID NO: 4, SEQ ID NO: 5, and SEQ ID NO: 6, respectively.

After cloning said protein variants in the vector pM238 as described, the proteins were obtained using two strategies: a) according to the process described by Morera et al., (Morera, et al., Angiogenesis, 2008: 11: 381-93), and b) according to the process described in Example 1. The resistance to trypsin digestion of the two purification variants was compared for each isoform. The analysis of the digestions was performed by molecular exclusion chromatography. The results indicated that, as described for PVM-I, when the variants of the VEGF-A isoforms are obtained according to the second purification strategy (PVM145-I, PVM165-I, PVM189-I, PVM206- I), resistance to trypsin digestion is observed in less than 5% of the total protein mass. This resistance was reflected in the presence of the signal corresponding to the dimer of the "cysteine knots", which is only obtained when the disulfide bridges are established according to the arrangement present in nature (signal between 23 and 25 minutes) (Table 9). Thus, when the protein preparations are obtained according to the process described in Example 1, they are more than 95% digested by incubation with trypsin, compared to the counterpart produced with the initial process described by Morera et al., as shown in Table 9. The peptides that are detailed in Table 3 (Example 1), and that confirm the absence of the canonical cysteine knot, were also confirmed by ESI-MS/ S for the digestions of the antigenic preparations PVM145-I, PVM165-I, PVM189-I, PVM206-I with trypsin, and could not be detected in the preparations PVM145, PVM165, PVM189, PVM206.

The comparative analysis of the immunogenicity of these variants, in Freund's adjuvant, was carried out as described in Example 1. This study showed a superior neutralization of the binding of VEGF-A to VEGFR2 by the sera of the animals treated with the variants with lower resistance to digestion with trypsin (PVM145-I, PVM165-I, PVM189-I, PVM206-I), when compared with its variants PVM145, PVM165, PVM189, PVM206 (Table 9).

**Table 9. Evaluation of the percent of protein recovery at 24.8 minutes after trypsin digestion of the antigenic variants. Analyses of the inhibition of VEGF-A binding to VEGFR2 by the serum of animals immunized with these variants.**

| **Proteic Preparation** | **Percentage of the total protein eluting at 24.8 minutes retention time (%)** | **Inhibition of VEGF-A-VEGFR2 (%)** |
|---|---|---|
| PVM145 | 95.8±0.9 | 62.1±2.8 |
| PVM145-I | 4.2±0.23 | **81.2±2.2** |
| PVM165 | 94.0±0.9 | 67.4±2.5 |
| PVM165-I | 3.7±0.43 | **90.1±1.2** |
| PVM189 | 85.3±0.9 | 60.8±5.4 |
| PVM189-I | 4.0±0.18 | **87.5±1.7** |
| PVM206 | 90.3±0.9 | 66.5±3.7 |
| PVM206-I | 5.0±0.18 | **92.4±2.6** |

These proteins were used in an immunization and dose schedule similar to that described in Example 5 in BALB/c animals challenged with 10000 CT26 cells by the subcutaneous route. The results indicated that 28 days after tumor inoculum, the use of antigenic variants with the adjuvant sNAcGM3-VSSP, induces a superior anti-tumor effect in the case of the variants PVM145-I, PVM165-I, PVM189-I, PVM206-I as compared to the preparations PVM145, PVM165, PVM189, PVM206 (Table 10).

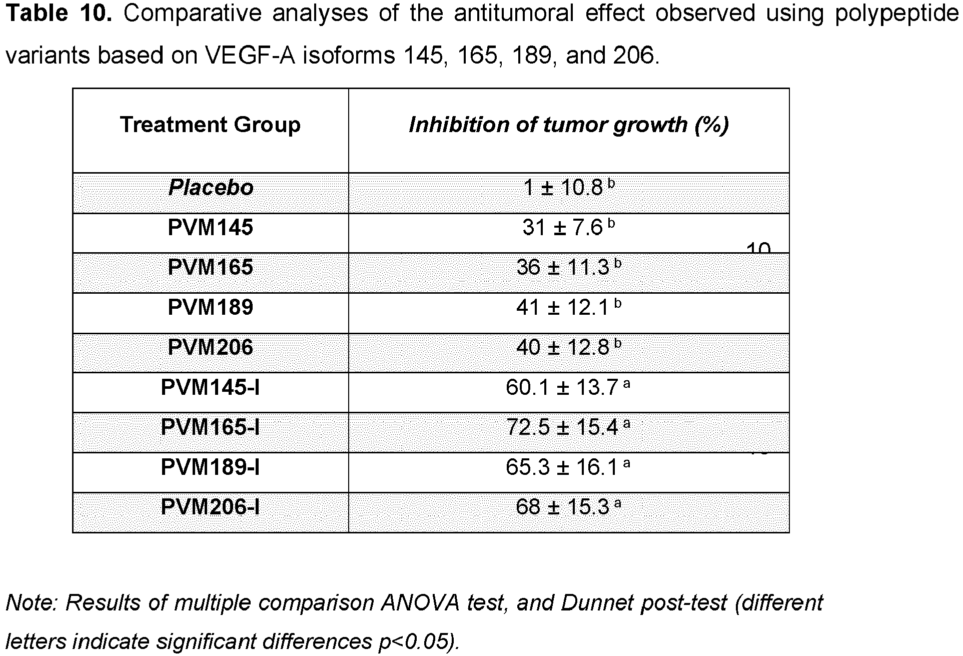

It was observed that, regardless of the VEGF-A isoform used, the variants that do not show resistance to digestion with trypsin are those with the highest immunogenicity and anti-tumor effect.

### Example 7. Preparation of PVM-I using oxidation and selection of trypsin sensitive variants.

Due to the formation of multiple disulfide bonds, several conformations coexist in the PVM-I protein preparation. These can be obtained and separated by processes that differ from that already described in Example 1, which include controlled denaturation and renaturation steps, in the presence of oxidizing agents. For the smaller isoform of VEGF-A expressed in E. coli, (SEQ ID NO: 2), the inclusion bodies were solubilized in 6M guanidium chloride, and the protein was purified by affinity to Nickel, in the presence of this Denaturing agent, using the manufacturer's recommendations (QIAGEN). The elution result was then separated in a reverse phase chromatography (RP), on a preparative C18 column, by a linear gradient between the buffers: (A) trifluoroacetic acid (TFA) in water (0.088% v/v) and (B) 0.084% TFA, 90% acetonitrile in water (v/v). In the fraction where the protein of interest eluted no resistance to digestion with trypsin was observed as expected, in agreement with the denatured and reduced state of the protein preparation. The protein was then subjected to an oxidative re-naturalization process in Tris-HCl pH 8.6; in the presence or not of guanidium chloride and β-mercaptoethanol (0.2 mM), for 20h at 22°C. After a buffer change to 10 mM Tris-HCl pH 7.4; by size exclusion chromatography on G25 sepharose; the formation of the disulfide bridges was evaluated. The formation of the "cysteine knot" was analyzed by digestion for 16h with Trypsin, in 200mM Tris-HCl buffer, pH 8.0, and at a 50:1 ratio (Protein: Trypsin), as described in Example 1. According to this the re-naturalized protein in the absence of guanidium chloride exhibited a 90% resistance to digestion (PVM-IA), while the re-naturalizations in the presence of the denaturing agent resulted in differential percentages of resistance to digestion, which decreased with increasing molarity of guanidium chloride.

With the use of 6M guanidium chloride, the highest number of fractions not resistant to digestion with trypsin was obtained, and all of them were called PVM-IO. These fractions were separated on RP-HPLC chromatography on a C18 preparative column. A total of 15 fractions were separated, which were numbered according to the elution order in the RP chromatography (F1 to F15, Table 11).

After a buffer exchange to 10 mM Tris pH 7.4; the PVM-IA, PVM-IO preparations, and the fractions of the latter were compared with PVM and PVM-I, in a study in BALB/c mice. The administration of proteins was evaluated in the conventional scheme of 8 administrations, one week apart, at a rate of 200 µg of the protein preparation in 100 µg of sNAcGM3-VSSP. Table 11 shows the results of the comparative analysis of the titers of specific IgG for murine VEGF-A, the inhibition of its binding to VEGFR2, the induction of a cytotoxic cellular response both in the lymph nodes and in the spleen; and the effect of immunization on subcutaneous CT26 tumor growth.

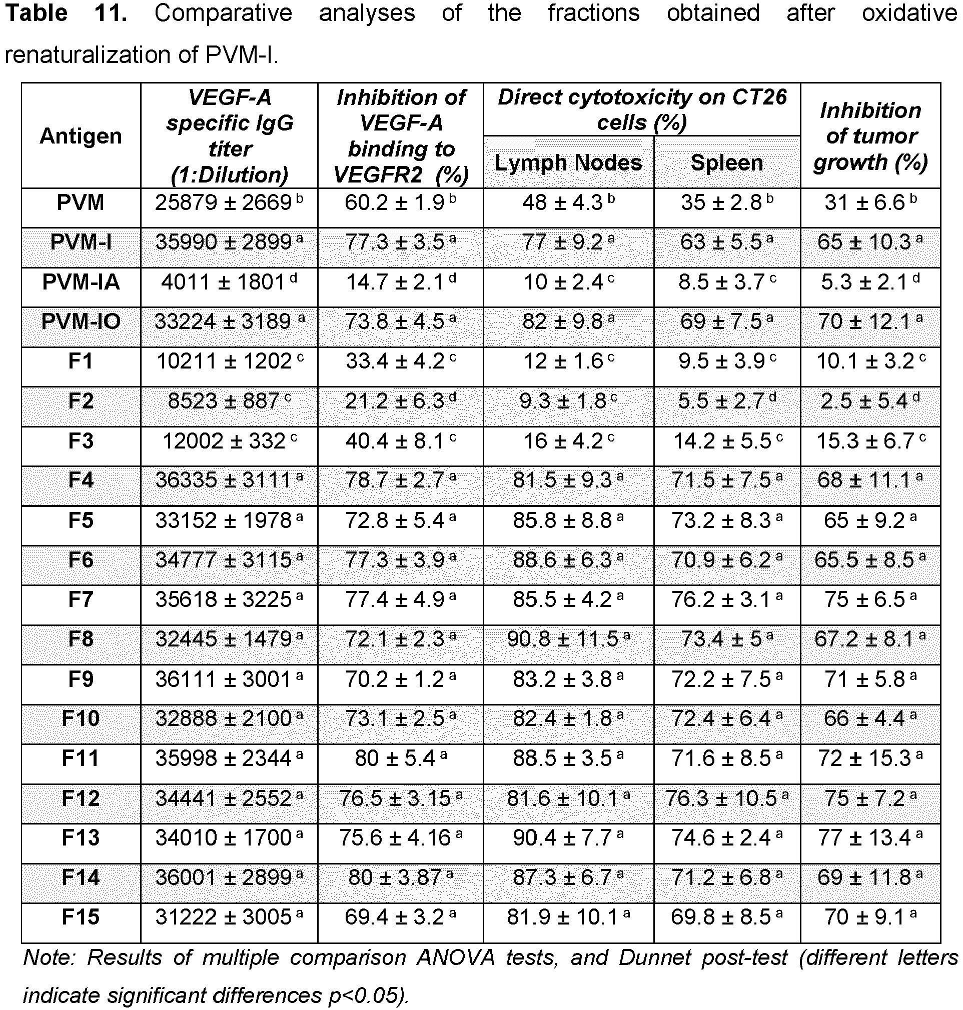

Table 11 shows the superior effect of the PVM-I preparation, as compared to the PVM preparation, which corroborates the result shown in Example 1 in Freund's adjuvant. This study illustrates the effects on immunity already described, and an anti-tumor effect that is directly related to the induction of an effective humoral and cellular response. The results of the analysis of the re-naturalized protein preparation in the absence of reducing agents (PVM-IA) indicate that it is between 7 and 10 times less immunogenic than PVM-I, and even than PVM, and results in a lower antitumor effect. However, with the use of the re-naturalized preparation in the presence of 6M guanidium chloride (PVM-IO), results are obtained that do not differ significantly from those described for PVM-I. Additionally, the evaluation of the fractions of the PVM-IO preparation indicates that at least 12 of these (F4-F15) are equipotent in the induction of a specific humoral and cellular immune response, as well as in the generation of an anti-tumor response. Analysis of these fractions by trypsin digestion and mass spectrometry, developed as described in Example 1, accompanied by an Edman degradation (Marti, T., Rosselet, SJ, Titani, K., and Walsh, KA (1987 ) Biochemistry 26, 8099-8109) allowed to verify that in fractions F4 to F15 cysteines 2 and 4 of the canonical structure of the cysteine knot are found forming intra-molecular bridges, while in the case of fractions F1 to F3 intermolecular junctions of either C2-C2, C4-C4, C2-C4, C2-C9, and C4-C9 are detected.

The ordering of the bonds in which the cysteines are involved in fractions F4 to F15 corresponds to the polypeptides identified in the sequence listing as SEQ ID NO: 18 to SEQ ID NO: 23, as detailed below:
- F4 corresponds to SEQ ID NO: 18, where the seventh cysteine (position 161) of two polypeptide chains, the eighth cysteine (position 163) of two polypeptide chains, and the last cysteine (position 175) of two polypeptide chains are forming intermolecular disulfide bridges.
- F5 corresponds to SEQ ID NO: 18, where the seventh cysteine (position 161) of a polypeptide chain with the eighth cysteine (position 163) of another polypeptide chain and the last cysteine of two polypeptide chains are forming intermolecular disulfide bridges.
- F6 corresponds to SEQ ID NO: 19, where the seventh cysteine (position 161) of two polypeptide chains, the eighth cysteine (position 163) of two polypeptide chains, and the last cysteine (position 175) of two polypeptide chains are forming intermolecular disulfide bridges.
- F7 corresponds to SEQ ID NO: 19, where the seventh cysteine (position 161) of a polypeptide chain with the eighth cysteine (position 163) of another polypeptide chain and the last cysteine of two polypeptide chains are forming intermolecular disulfide bridges.
- F8 corresponds to SEQ ID NO: 20, where the seventh cysteine (position 161) of two polypeptide chains, the eighth cysteine (position 163) of two polypeptide chains, and the last cysteine (position 175) of two polypeptide chains are forming intermolecular disulfide bridges.
- F9 corresponds to SEQ ID NO: 20, where the seventh cysteine (position 161) of a polypeptide chain with the eighth cysteine (position 163) of another polypeptide chain and the last cysteine of two polypeptide chains are forming intermolecular disulfide bridges.
- F10 corresponds to SEQ ID NO: 21, where the seventh cysteine (position 161) of two polypeptide chains and the eighth cysteine (position 163) of two polypeptide chains are forming intermolecular disulfide bridges.
- F11 corresponds to SEQ ID NO: 21, where the seventh cysteine (position 161) of a polypeptide chain with the eighth cysteine (position 163) of another polypeptide chain are linked, forming intermolecular disulfide bridges.
- F12 corresponds to SEQ ID NO: 22, where the seventh cysteine (position 161) of two polypeptide chains, and the eighth cysteine (position 163) of two polypeptide chains, are linked forming intermolecular disulfide bridges.
- F13 corresponds to SEQ ID NO: 22, where the seventh cysteine (position 161) of a polypeptide chain with the eighth cysteine (position 163) of another polypeptide chain are linked forming intermolecular disulfide bridges.
- F14 corresponds to SEQ ID NO: 23, where the seventh cysteine (position 161) of two polypeptide chains, and the eighth cysteine (position 163) of two polypeptide chains, are linked forming intermolecular disulfide bridges.
- F15 corresponds to SEQ ID NO: 23, where the seventh cysteine (position 161) of a polypeptide chain with the eighth cysteine (position 163) of another polypeptide chain are linked forming intermolecular disulfide bridges.

### Example 8. Analysis of the relevance of Cysteines 2 and 4 in the generation of immunogenic structures.

The results described in Examples 1, 9, and 10 indicate that a feature common to the more immunogenic structures of PVM-I is the fact that the intramolecular linkages in VEGF-A involve cysteines 2 and 4 of the primary sequence (Cys 110 and Cys 119 in PVM-I). These residues appear mutated with a high frequency in the studies reviewed to obtain VEGF-A since this avoids the dimeric aggregation of the protein (Jiang, et al., Biochemistry, 2010: 49: 6550-6, Wentink, et al., Proc Natl Acad Sci USA, 2016: 113: 12532-7). In the natural conformation, these are the amino acids that form the interchain bridges to generate the VEGF-A dimer. Only the previous works by Bequet-Romero et al., and Morera et al., Use the primary sequence variant of VEGF-A that includes these cysteines, however in none of them the formation of intra-chain disulfide bonds between cysteines 2 and 4 of the sequence where observed. The presence of these amino acids in the sequence used to generate the antigen and the process by which they are obtained so that the cysteines mentioned form only intra-chain bonds, constitute a novel approach to obtaining the protein for vaccination purposes. To better characterize the relevance of these residues for the immunogenicity of the antigenic preparation, we proceeded to obtain (by site-directed mutagenesis) the variants that include the following changes from cysteine to alanine in the primary sequence:
a) PVM-I^{Ala110}: Cys110 by Ala110
b) PVM-I^{Ala119}: Cys119 by Ala119
c) PVM-I^{Ala110}, 119: Cys110 for Ala110 and Cys119 for Ala119

For this purpose, the oligonucleotide pairs indicated in Table 12 were used to introduce the mutations through a nested polymerase chain reaction (PCR), as described (Bequet-Romero, et al., Angiogenesis, 2007: 10: 23-34)). Briefly, PCRs 1 and 2 were performed, taking as a template the plasmid containing the sequence coding for SEQ ID NO: 2. The mixture for PCR MasterMix (Qiagen) and the oligonucleotides indicated in Table 12, were used. Amplified DNA in each reaction was purified after separation on agarose gels from the reaction template. The two resulting DNA strands were used as a template in PCR3 for each molecule described in Table 12. A 25-cycle PCR was performed and the resulting DNA, after being separated on agarose gels, was digested with the enzymes Nhel and BamHI (Promega), according to the manufacturer's instructions. The digested DNA was cloned into the vector pM238, as described (Morera, et al., Angiogenesis, 2008: 11: 381-93). Automatic sequencing of the resulting DNA verified the introduced mutations. The DNA encoding these mutated proteins were transformed into strain BL21 (DE3), and the polypeptides were produced according to the process described in Example 1 for PVM-I. The polypeptides corresponding to the sequences SEQ ID NO: 15, SEQ ID NO: 16, and SEQ ID NO: 17 results in the preparations PVM-I^{Ala110}, PVM-I^{Ala119}, and PVM-I^{Ala110,119}.

**Table 12. Oligonucleotides that were used in the PCR reactions to obtain the PVM-I mutants.**

| | **Oligonucleotides for PCR 1** | **Oligonucleotides for PCR 2** | **Oligonucleotides for PCR 3** |
|---|---|---|---|
| **PVM-I^{Ala110}** | SEQ ID NO: 13, SEQ ID NO: 7 | SEQ ID NO: 8, SEQ ID NO: 14 | SEQ ID NO: 13, SEQ ID NO: 14 |
| **PVM-I^{Ala119}** | SEQ ID NO: 13, SEQ ID NO: 9 | SEQ ID NO: 10, SEQ ID NO: 14 | SEQ ID NO: 13, SEQ ID NO: 14 |
| **PVM-I^{Ala110,119}** | SEQ ID NO: 13, SEQ ID NO: 11 | SEQ ID NO: 12, SEQ ID NO: 14 | SEQ ID NO: 13, SEQ ID NO: 14 |

The preparations containing the proteins with mutations in cysteines 110 and 119 were compared with PVM and PVM-I, in a study in BALB/c mice, where their administration was evaluated in the scheme of 8 administrations, separated by intervals of one week, at a rate of 200 µg of the antigenic preparation in 100 µg of the adjuvant sNAcGM3-VSSP. The comparative study was carried out in the subcutaneous CT26 colon carcinoma model, with 10 animals per group. Table 13 shows the superior immunogenicity of the PVM-I preparation compared to the PVM, which corroborates the result shown in Example 1. The results of the analysis of the protein preparations of the mutants in cysteines 110 and 119 indicate that they are approximately 2 times less immunogenic than PVM-I, and even PVM, and result in a lower anti-tumor effect.

**Table 13. Comparative analysis of the antigenic preparations PVM, PVM-I, PVM-IAla110, PVM-I^{Ala119}, and PVM-I^{Ala110,119}.**

| **Antigen** | ***Inhibition VEGF-A*/ *VEGFR2 binding (%)*** | ***Direct celular response on CT26 cells (%)*** | | ***Inhibition of tumor growth (%)*** |
|---|---|---|---|---|
| | | **Linfonodos** | **Bazo** | |
| **PVM** | **60.2 ± 1.9 ^{b}** | **50.2 ± 9.3 ^{b}** | **32.3 ± 4.8 ^{b}** | **39 ± 6.6 ^{b}** |
| **PVM-I** | **77.3 ± 3.5 ^{a}** | **72.5 ± 11.2 ^{a}** | **65.1 ± 6.5 ^{a}** | **60 ± 10.3 ^{a}** |
| PVM-I^{Ala110} | **30.3 ± 7.1 ^{c}** | **17 ± 4.9 ^{c}** | **19.3 ± 6.1 ^{c}** | **22.3 ± 8.5 ^{c}** |
| PVM-I^{Ala119} | **43.1 ± 10.5 ^{c}** | **22 ± 5.8 ^{c}** | **21.5 ± 7.1 ^{c}** | **27.3 ± 7.4 ^{c}** |
| PVM-I^{Ala110.119} | **28.9 ± 4.8 ^{c}** | **25 ± 3.1 ^{c}** | **17.5 ± 3.4 ^{c}** | **21.1 ± 3.2 ^{c}** |

| | | | | |
|---|---|---|---|---|
| *Note: Results of multiple comparison ANOVA tests, and Dunnet post-test (different letters indicate significant differences p<0.05).* | | | | |

### Example 9. Comparative evaluation of the preclinical use of the antigenic preparations PVM and PVM-I in the treatment of lung metastases in mice.

Metastases are the fundamental cause of death from cancer of any origin. Of these, those located in the lungs are the ones that most frequently lead to the death of patients, due to the respiratory complications they cause, due to the replacement of lung tissue by the tumor, and the accumulation of pleural fluid. Metastases in the lung constitute one of the most resistant situations for anti-angiogenic interventions based on the single administration of agents that eliminate the ligand or signal suppressors that transduce VEGF-A receptors. In this context, the incorporation of the cellular branch of the immune response, to the arsenal of response to immunization with vaccines, could play a decisive role.

To evaluate the efficacy of the vaccine preparations under analysis, for the treatment of lung metastases in mice, groups of 12 mice were immunized with these preparations, and the tumor challenge was performed according to the requirements of every model. The results of immunization with sNAcGM3-VSSP as adjuvant are described below for three metastasis models. The animals were immunized for 8 weeks, at a rate of a weekly dose of 200 µL of total volume, subcutaneously, containing 100 µg of the corresponding antigen and 100 µg of the adjuvant.

For the study in the mouse strain C57BI/6, 250,000 cells of the 3LL metastatic lung carcinoma, clone D122, were inoculated into the footpad 3 days after the fourth immunization. The primary tumor was removed by surgery, 20 days after implantation, and the animals were sacrificed 15 days later to count and characterize the lung metastases. The analysis of the metastatic load was assessed by evaluating the lung's weight and the number of macrometastases. The mitotic index study was carried out by measuring the number of mitoses per field, in 10 fields per cut, per animal. Likewise, the apoptotic figures were quantified per field and the Mitosis / Apoptosis ratio was calculated.

Table 14 shows the comparison of the lung weights of the animals subjected to the treatments. A differential effect was observed between both antigens, which favored the antigenic preparation PVM-I, enriched in the family of structural isomers that completely lack the disulfide bonds present in natural VEGF-A. The histopathological characterization of the metastatic lesions, using hematoxylin-eosin staining, and the analysis of serial sections, showed that the specific immune response to VEGF-A also translates into a significant reduction of the mitosis/apoptosis balance observed in the metastatic foci.

**Table 14. Effect of the administration of the antigenic preparations PVM and PVM-I in the model of spontaneous metastasis to the lung of the subcutaneous tumor 3LL-D12**

| **Treatment** | **Lung Weight (g)** | **Number of macrometastases** | **Mitosis/Apoptosis ratio** |
|---|---|---|---|
| No Treatment | 0.6562 ± 0.336 *^{a}* | 7.727 ± 6.574 *^{a}* | 2.533 ± 0.9415 *^{a}* |
| PVM in sNAcGM3-VSSP | 0.3975 ± 0.11 *^{b}* | 4.273 ± 4.027 *^{b}* | 1.52 ± 0.5993 *^{b}* |
| PVM-I in sNAcGM3-VSSP | 0.279 ± 0.039 *^{c}* | 1.333 ± 1.614 *^{c}* | 0.8731 ± 0.1439 *^{c}* |
| Excipients in sNAcGM3-VSSP | 0.498 ± 0.167 *^{a}* | 9.818 ± 5.382 *^{a}* | 2.881 ± 0.7743 *^{a}* |

| | | | |
|---|---|---|---|
| *Note: Results of multiple comparison ANOVA tests, and Dunnet post-test (different letters indicate significant differences p<0.05).* | | | |

In the mouse strain BALB/c, the establishment of experimental lung metastases was evaluated after intravenous inoculation of 20,000 CT26 carcinoma cells. The challenge was performed through the retro-orbital plexus, three days after the fourth immunization. The animals were sacrificed 30 days after the tumor challenge, and the tumor burden in the lungs, as well as the morphology, cell density, angiogenesis, and the mitosis/apoptosis balance, were evaluated in the histopathological analysis of the serial sections of the lung tissue. As shown in Table 15, both the number of metastases and the area they occupy in the analyzed sections (10 per animal), is reduced with immunization with PVM. The most significant effect was observed when immunizing with the PVM-I preparation (p <0.05; Dunnet's post-test). Interestingly, the mitotic index (number of mitoses per field), which is very high for metastatic lesions in the lung caused by this model, was reduced approximately 4 times when the vaccine preparation was administered. As in the previous analysis, the best effects were observed in the group treated with the antigenic variant PVM-I.

**Table 15. Effect of the administration of the antigenic preparations PVM and PVM-I in the model of experimental metastasis to the lung after intravenous administration of CT26 colon carcinoma.**

| **Treatment** | **Number of macro metastases** | **Mitosis/Apoptosis Ratio** |
|---|---|---|
| No Tratamiento | 16.22 ± 8.913 *^{c}* | 5.567 ± 1.197 *^{a}* |
| PVM in sNAcGM3-VSSP | 7.778 ± 6.797 *^{b}* | 3.244 ± 1.638 *^{b}* |
| PVM-I in sNAcGM3-VSSP | 1.889 ± 2.977 *^{a}* | 1.8 ± 0.6325 *^{c}* |
| Excipients in sNAcGM3-VSSP | 22.22 ± 10.4 *^{c}* | 6.422 ± 2.524 *^{a}* |

| | | |
|---|---|---|
| *Note: Results of multiple comparison ANOVA tests, and Dunnet post-test (different letters indicate significant differences p<0.05).* | | |

In the BALB/c mouse strain, the F3II metastatic breast carcinoma model, which spontaneously metastasizes to the lung, was also evaluated. In this case, the use of the biweekly scheme was included, with aluminum phosphate adjuvant as an additional group. Three days after the fourth immunization (in the presence of sNAcGM3-VSSP) or the second immunization (in the presence of aluminum phosphate), a total of 200 000 cells were inoculated, in 100 µL of DMEM culture medium, subcutaneously.

Table 16 shows the analysis of the weight of the primary tumor, 28 days after tumor implantation. Treatment of the animals with both antigenic variants, and in both adjuvants and administration schemes, significantly reduces tumor growth, but it is the antigenic preparation PVM-I that shows a more significant inhibition (p <0.05, Dunnet's post-test). The same table shows the results of the lung macrometastases count. As can be seen, the reduction in tumor growth translates into a significant reduction in the number of metastases that are implanted. Besides, in these studies, a reduction in the mitosis/apoptosis balance was observed, and an increase in the number of necrotic foci associated with the metastatic lesions detected. All these effects are detected with greater significance in the group treated with the antigenic preparation PVM-I.

**Table 16. Effect of the administration of the antigenic preparations PVM and PVM-I in two adjuvants in the F3II metastatic breast carcinoma model.**

| | **Tumor Growth** | **Number of Metastases** |
|---|---|---|
| **Treatment** | **n=10** | **n=12** |
| No Treatment | 3.375 ± 0.3701 *^{a}* | 18 ± 3.643 *^{a}* |
| PVM in sNAcGM3-VSSP | 2.231 ± 0.5614 *^{b}* | 7.25 ± 3.306 *^{b}* |
| PVM-1 in sNAcGM3-VSSP | 1.579 ± 0.4538 *^{c}* | 2.417 ± 1.621 *^{c}* |
| Excipients in sNAcGM3-VSSP | 3.502 ± 0.4347 *^{a}* | 21.08 ± 4.907 *^{a}* |
| PVM in Alum Phosphate | 2.387 ± 0.5571 *^{b}* | 9.167 ± 3.614 *^{b}* |
| PVM-1 in Alum Phosphate | 1.691 ± 0.3536 *^{c}* | 3.25 ± 2.094 *^{c}* |
| Excipients in Alum Phosphate | 3.508 ± 0.5316 *^{a}* | 21.50 ± 4.075 *^{a}* |

| | | |
|---|---|---|
| *Note: Results of multiple comparison ANOVA tests, and Bonferroni post-test (different letters indicate significant differences p<0.05).* | | |

Overall, the results indicated that the antigenic preparation PVM-I was more effective in reducing the metastatic potential of primary tumors, and the implantation capacity of metastatic cells in the lung. Furthermore, a superior effect of vaccination with the antigenic preparation PVM-I was observed in the change of the metastatic phenotype, observing cell foci with a reduced mitosis/apoptosis balance, a higher incidence of necrosis, and a significant reduction in angiogenesis.

### Example 10. Effect of immunization with antigenic preparations PVM and PVM-I on tumor-induced immunosuppression.

Groups of 15 animals of the BALB/c strain were used, which were immunized subcutaneously with the following variants:
1. PVM in sNAcGM3-VSSP (8 immunizations, weekly frequency)
2. PVM-I in sNAcGM3-VSSP (8 immunizations, weekly frequency)
3. sNAcGM3-VSSP (8 immunizations, weekly frequency)
4. PVM in aluminum phosphate (4 immunizations, bi-weekly frequency)
5. PVM-I in aluminum phosphate (4 immunizations, bi-weekly frequency)
6. Aluminum phosphate (4 immunizations, bi-weekly frequency)

In all cases, immunization was performed subcutaneously, with a total volume of 200 µL. Three days after the last immunization, 5 animals per group were randomly selected and euthanized, to analyze their immunological status and that of the experimental controls (Groups 3 and 6). This evaluation was performed by flow cytometry, in whole blood samples, in spleen cells, lymph nodes, and bone marrow.

The remaining animals in each group receive a subcutaneous challenge with 20 000 CT26 colon carcinoma cells on the right flank. At 14 and 21 days after the injection of tumor cells, five mice per group were euthanized and evaluated, adding, in this case, the analysis of dissociated tumor tissue.

No occurrence of toxic events at a macroscopic level was evidenced for any animal, and the histopathological analysis did not reveal the presence of damage in any of the organs analyzed 7 days after the last immunization. The immunological evaluation consisted of (1) the analysis of the serum concentration of murine VEGF-A; (2) evaluation of the concentration of T lymphocytes in whole blood, lymph nodes draining the primary tumor and the tumor itself; (3) the study of IFN-gamma secretion by intratumoral lymphocytes when exposed to a representative antigen of mutated VEGF-A.

In non-treated animals murine VEGF-A levels increase with the time of exposure to the tumor in agreement with the increase of tumor size. In the groups immunized with the antigenic variants, a significant reduction (p <0.001 ANOVA, Dunnet's post-test) was observed in VEGF-A levels, which lasted even 30 days after the tumor challenge, (Table 17). Towards the tumor compartment, a similar phenomenon was observed.

**Table 17. Análisis de las concentraciones de VEGF-A murino en el suero y en el lisado tumoral de los animales inmunizados.**

| **Group** | **Treatment** | **VEGF-A (pg/mL) in serum** | | **VEGF-A (pg/mL) in tumor** | |
|---|---|---|---|---|---|
| | | **Day 14** | **Day 21** | **Day 14** | **Day 21** |
| I | PVM in sNAcGM3-VSSP | 75. 5 ± 26 ^{b} | 110.2 ± 15 ^{b} | 137 ± 34 ^{b} | 250.2 ± 38 ^{b} |
| II | PVM-I in sNAcGM3-VSSP | 52.2 5 ± 23 ^{c} | 62.5 ± 12 ^{c} | 88.7 ± 27 ^{c} | 103.1 ± 11 ^{c} |
| III | sNAcGM3-VSSP | 116.5 ± 50 ^{a} | 183.4 ± 73 ^{a} | 304.4 ± 31 ^{a} | 516.1 ± 20 ^{a} |
| IV | PVM in aluminum phosphate | 85.1 ± 16 ^{b} | 108.7 ± 18 ^{b} | 161.2 ± 37 ^{b} | 293 ± 30 ^{b} |
| V | PVM-I in aluminum phosphate | 49.5 ± 19 ^{c} | 76.3 ± 21 ^{c} | 103.4 ± 24 ^{c} | 140.9 ± 20 ^{c} |
| VI | Aluminum Phosphate | 120.3 ± 35 ^{a} | 207.6 ± 62 ^{a} | 420.1 ± 63 ^{a} | 643.4 ± 87 ^{a} |
| VII | No treatment | 106.2 ± 50 ^{a} | 195.15 ± 73 ^{a} | 387.4 ± 43 ^{a} | 601.2 ± 92 ^{a} |
| VIII | No treatment / No tumor | 30 ± 5 | 27 ± 11 | | |

| | | | | | |
|---|---|---|---|---|---|
| *Note: Results of multiple comparison ANOVA tests, and Bonferroni post-test (different letters indicate significant differences).* | | | | | |

The state of the immune system of the sacrificed animals, at each moment, was analyzed through the study of the proportions of the cell populations, present in the lymph nodes and the tumor itself, as reported by Gabrilovich et al., (Gabrilovich D et al. Blood 1998, 92: 4150). For these studies, monoclonal antibodies against CD3, CD4, CD8, labeled with fluorescein isothiocyanate, phycoerythrin, and Phycoerythrin-Cy7, were used, which allowed the visualization of the cell populations using a flow cytometer (Sysmex Partec). The results obtained are reflected in Table 18.

**Table 18. Summary of the results of the analysis of the cell populations of interest.**

| **WB** | **Grupo** | **CD3** | **CD4** | **CD8** | |
|---|---|---|---|---|---|
| I | PVM in sNAcGM3-VSSP | 3260 ± 125 | 2341 ± 140 | 789 ± 63 | |
| II | PVM-I in sNAcGM3_VSSP | 3890 ± 446 | 2652 ± 236 | 897 ± 115 | |
| III | sNAcGM3-VSSP | 1267± 181 | 1267± 181 | 212 ± 58 | |
| IV | PVM in Alum Phosphate | 2950 ± 185 | 2950 ± 185 | 690 ± 94 | |
| V | PVM-I in Alum Phosphate | 3575 ± 283 | 3575 ± 283 | 795 ± 106 | |
| VI | Alum Phosphate | 1320 ± 230 | 1320 ± 230 | 198 ± 63 | |

| **DLN** | **Grupo** | **CD3** | **CD4** | **CD8** | |
|---|---|---|---|---|---|
| I | PVM in sNAcGM3-VSSP | 123663 ± 3548 | 78898 ± 2689 | 32354 ± 1779 | |
| II | PVM-I in sNAcGM3_VSSP | 166621 ± 6652 | 90003 ± 3223 | 45000 ± 2600 | |
| III | sNAcGM3-VSSP | 90114 ± 1052 | 58454 ± 1290 | 24241 ± 2003 | |
| IV | PVM in Alum Phosphate | 114423 ± 2848 | 68878 ± 3672 | 30245 ± 2158 | |
| V | PVM-I in Alum Phosphate | 156771 ± 5542 | 81123 ± 3117 | 43222 ± 3485 | |
| VI | Alum Phosphate | 80333 ± 1242 | 60343 ± 1450 | 19541 ± 1253 | |

| **T** | **Grupo** | **CD3** | **CD4** | **CD8** | **% CD8 PD1⁻** |
|---|---|---|---|---|---|
| I | PVM in sNAcGM3-VSSP | 4542 ±145 | 2525 ± 253 | 3400 ± 350 | 75 ± 5.1 |
| II | PVM-I in sNAcGM3_VSSP | 6995 ± 230 | 2855 ± 125 | 3985 ± 180 | 92 ± 3.1 |
| III | sNAcGM3-VSSP | 2538 ± 93 | 1240 ± 52 | 1418 ± 277 | 50 ± 4.5 |
| IV | PVM in Alum Phosphate | 3542 ± 152 | 1525 ± 98 | 2400 ± 84 | 63 ± 4.2 |
| V | PVM-I in Alum Phosphate | 6583 ± 302 | 2514 ± 96 | 3408 ± 102 | 72 ± 2.4 |
| VI | Alum Phosphate | 2597 ± 212 | 1025 ± 98 | 1610 ± 201 | 42 ± 3.7 |

| | | | | | |
|---|---|---|---|---|---|
| *Note: Whole Blood (WB), Draining Lymph Nodes (DLN), and subcutaneous tumor (T).* | | | | | |

The analyzes of the lymphoid cell populations in the animals, 14 days after the tumor challenge, show an increase in the CD3, CD4, and CD8 positive cell fractions, in the three analyzed compartments, which is directly related to the administration of the vaccine preparations. With the antigenic variant PVM-I, significantly higher concentrations of these cells of the immune system are achieved (p<0.05 Bonferroni post-test). Additionally, the study of the PD1 marker in CD8 T lymphocytes, infiltrated in the subcutaneous tumor, indicated that the increase of these was associated with a negative PD1 phenotype, in those animals treated with the antigens, in a higher percentage in the case of the who received the PVM-I antigen (Table 18). The increase in the infiltrate of CD4 and CD8 cells was positively and significantly related to a reduction in tumor mass (r = 0.7147, p <0.002).

Leukocytes from the tumor were isolated by a positive selection process, in columns, using magnetic beads coated with an antibody specific for CD45 (Miltenyi), and IFN-gamma secretion was evaluated in response to the addition of VEGF -A mutated in the culture medium, using an ELISPOT type system (MABTECH). Only in the case of animals vaccinated with the antigen was an increase in the secretion of this cytokine observed, and it was higher for both adjuvants, in the group that received the PVM-I antigen (p <0.05, post-test Bonferroni) (Table 19). This showed that not only cell infiltration was increased, but that these infiltrating leukocytes had a higher activity associated with the administration of the vaccine preparations.

**Table 19. Analysis of the tumor CD45 + infiltrates by IFN-gamma ELISPOT.**

| | **Group** | **Number of IFN-gamma secretor clones/ 10⁶ CD45⁺ cells** |
|---|---|---|
| I | PVM in sNAcGM3-VSSP | 120 ± 34^{c} |
| II | PVM-I in sNAcGM3_VSSP | 352 ± 130^{a} |
| III | sNAcGM3-VSSP | 42 ± 14^{d} |
| IV | PVM in Alum Phosphate | 105 ± 23^{c} |
| V | PVM-I in Alum Phosphate | 203 ± 102^{b} |
| VI | Alum Phosphate | 32 ± 12^{d} |

### Example 11. Evaluation of the adjuvant effect of immunization with PVM-I on the immunogenicity that is generated to another antigenic challenge.

Given its potential for immune restoration, it was explored whether immunization with PVM-I could induce an adjuvant effect with an increased humoral and cellular response specific not only for VEGF-A but also for an unrelated antigen. This potential effect was examined by evaluating the extent of specific immunity to antigens present in the tumor and to antigens that are co-administered with the vaccine.

The ovarian tumor model ID8-OVA was used for the tumor challenge of the C57BI/6 animals and the parental line ID8 in the in vitro lysis studies. The animals (n=10 per group) received a tumor challenge of 2.5 million cells, intraperitoneally, and three days later the immunization schedules started, as described below:
a) PVM-I (200 µg), sNAcGM3-VSSP (100 µg): weekly subcutaneous administration, for 8 weeks
b) OVA (1 mg), sNAcGM3-VSSP (100 µg): weekly subcutaneous administration, for 2 weeks. sNAcGM3-VSSP (100 µg): weekly subcutaneous administration for the remaining 6 weeks
c) PVM-I (200 µg), sNAcGM3-VSSP (100 µg), OVA (1mg): weekly subcutaneous administration for 2 weeks and PVM-I (200 µg), sNAcGM3-VSSP (100 µg): weekly subcutaneous administration for the remaining 6 weeks.
d) sNAcGM3-VSSP (100 µg): weekly subcutaneous administration for 8 weeks
e) OVA (1 mg): weekly subcutaneous administration for 2 weeks. sNAcGM3-VSSP (100 µg): weekly subcutaneous administration for the remaining 6 weeks.

One week after the eighth immunization, and 60 days after tumor implantation, the animals were euthanized. In this tumor model, the weight of the animals correlates with the growth of the tumor. Table 20 shows the observed anti-tumor effect, which is cooperatively increased by administering OVA in the presence of PVM-I in sNAcGM3-VSSP.

The analysis of the humoral response for VEGF-A and OVA was carried out using ELISA-type systems in the collected sera, as described in Examples 1, 2, and 3. The specific cellular response was analyzed using specific cell lysis studies tumor, by in vitro co-incubation of spleen cells with ID8 and ID8-OVA tumor cell lines (as described in Example 4). Furthermore, the secretion of IFN-gamma in the supernatant of spleen cells isolated from the immunized animals was examined, in response to the addition of the specific stimuli for 72 h (peptide OVA ₂₅₇₋₂₆₄, or VEGF_{KDR}-). In the latter case, an ELISA type assay was used for the detection of IFN-gamma, according to the manufacturer's instructions (Biolegend). The results are summarized in Tables 20 and 21.

**Table 20. Weight of the animals at the end of the study and specific IgG titers for VEGF-A and OVA antigens.**

| **Group** | **Weight (g)** | **Titer vs VEGF-A** | **Titer vs OVA** |
|---|---|---|---|
| **PVM-I/NAcGM3-VSSP** | 24,90 ± 0,7 ^{b} | 10580 ± 2056 ^{a} | 3800 ± 152 ^{c} |
| **OVA/NAcGM3-VSSP** | 25,76 ± 1,4 ^{b} | 135 ± 22 ^{b} | 10080 ± 1202 ^{b} |
| **PVM-I/NAcGM3- VSSP/OVA** | 23,37 ± 1,7 ^{c} | 10320 ± 1842 ^{a} | 20955 ± 1242 ^{a} |
| **NAcGM3-VSSP** | 27,83 ± 2,5 ^{a} | 200 ± 76 ^{b} | 140 ± 24 ^{d} |
| **OVA** | 29,13 ± 3,8 ^{a} | 180 ± 54 ^{b} | 200 ± 52 ^{d} |

| | | | |
|---|---|---|---|
| *Note: Results of multiple comparison ANOVA tests, and Dunnet post-test (different letters indicate significant differences p<0.05).* | | | |

**Table 21. Comparative analyses of the results of the cellular response.**

| **Group** | **IFN-gamma VEGF_{KDR}-** | **IFN-gamma OVA ₂₅₇₋₂₆₄** | **CTL-ID8 (%)** | **CTL-ID8-OVA (%)** |
|---|---|---|---|---|
| **PVM-I/NAcGM3-VSSP** | **1650 ± 98^{a}** | **1200 ± 54^{c}** | **35 ± 3^{a}** | **33 ± 5.3^{b}** |
| **OVA/NAcGM3-VSSP** | 10 ± 25^{b} | **3045 ± 39^{b}** | 5 ± 2^{b} | **28± 6.1^{b}** |
| **PVM-I/NAcGM3- VSSP/OVA** | **1700 ± 120^{a}** | **3800 ± 152^{a}** | **35 ± 3^{a}** | **79 ± 4.1^{a}** |
| **NAcGM3-VSSP** | 90 ± 45^{b} | 59 ± 26^{d} | 5 ± 2.1^{b} | 6 ± 1.5^{c} |
| **OVA** | 25 ± 23^{b} | 100 ± 44^{d} | 6.5 ± 3^{b} | 15 ± 3.4^{c} |

| | | | | |
|---|---|---|---|---|
| *Percentages indicate the lysis of ID8 or ID8-OVA cells in the final point of the study. Results of multiple comparison ANOVA tests, and Dunnet post-test (different letters indicate significant differences p<0.05).* | | | | |

The humoral (Table 20) and the cellular (Table 21) responses are induced for both antigens when administered in the context of sNAcGM3-VSSP. The co-administration of OVA with immunotherapy that includes PVM-I leads to a significant increase in the specific response to OVA, not only in animals that received OVA as an immunogen but also in those that only received PVM-I. This fact indicates the potentiality of the vaccine strategy with this antigenic preparation in the induction of an amplified response to antigens that are not found within the vaccine preparation, but that is expressed by the tumor. The humoral response, as well as IFN-gamma secretion and ID8-OVA cell lysis, in the group treated with both antigens, indicates that the combination not only has an additive effect but that there is a cooperation that induces a superior effect. This element indicates the possibilities of using this strategy in increasing the immune response to other tumor-associated antigens.

### Example 12. In vivo protection experiments in the collagen-induced arthritis model by immunization with PVM or PVM-I in two adjuvants.

Groups of 20 DBA/1 mice (H-2q haplotype) were immunized, which are susceptible to collagen-induced arthritis. Animals received the investigational antigenic preparations (PVM or PVM-I) adjuvanted in aluminum phosphate or sNAcGM3-VSSP. For sNAcGM3-VSSP, the scheme of 8 doses of 100 µg of antigen and 100 µg of adjuvant, with weekly intervals, was used, and in the case of aluminum phosphate, 4 doses were administered, at bi-weekly intervals, of 100 µg of antigen in 0.7 mg equivalent of Al ³⁺. Treatment groups were defined as follows:
I. PVM in sNAcGM3-VSSP
II. PVM-I in sNAcGM3-VSSP
III. sNAcGM3-VSSP
IV. PVM in Aluminum Phosphate
V. PVM-I in Aluminum Phosphate
VI. Aluminum phosphate

Three days after the fourth immunization with NAcGM3-VSSP or the second immunization with aluminum phosphate, the induction of autoimmune arthritis by immunization with chicken collagen II (Sigma) was initiated, according to the previously described model (Campbell IK et al Eur. J. Immunol. 30: 1568, 2000). This immunization was repeated on day 26, to complete the induction of the autoimmune response. The four legs of the mice were evaluated daily, according to the arthritis index, which establishes a score of 0 to 3 for each leg, for the presence in the examination of signs of erythema (1), inflammation (2), or joint stiffness (3), with a maximum value of 12. The mice began to manifest the clinical symptoms of the development of arthritis 23 days after induction, reaching the maximum incidence at 50 days. Table 22 shows the analysis of the incidence of arthritis in the animals of the different experimental groups. On days 40 and 55, a significant reduction in the incidence of arthritis was observed in the vaccinated groups (I, II, IV, and V), compared to the control groups III and VI, which received the placebo in the corresponding adjuvant.

**Table 22. Incidence of arthritis in two evaluation moments**

| **Group** | **Incidence by day 40** | **Incidence by day 55** |
|---|---|---|
| I | 20/7 (35%) | 20/9 (45%) |
| II | 20/4 (20%) | 20/6 (30%) |
| III | 20/12 (60%) | 20/16 (80%) |
| IV | 20/8 (40%) | 20/12 (60%) |
| V | 20/6 (30%) | 20/8 (40%) |
| VI | 20/13 (65%) | 20/15 (75%) |

### Example 13. Evaluation of the immunogenicity of PVM and PVM-I in non-human primates and analysis of the effect of immunization on experimental laser-induced choroidal neovascularization.

The ability of the antigenic preparations PVM and PVM-I to induce a relevant immune response, in a more autologous context, was evaluated in non-human primates (Chlorocebus aethiops sabaeus). 4 animals were used per group. 400 µg of the antigenic preparation PVM or PVM-I was administered, in 200 µg of NAcGM3-VSSP or 0.7 mg of Al ³⁺ equivalents in the form of aluminum phosphate. The administration schedules were 8 weekly doses in the case of NAcGM3-VSSP, and 4 biweekly doses for aluminum phosphate. The specific IgG antibody titers for VEGF-A, and the neutralization of its binding to its type 2 receptor, was evaluated at week 8 of immunization (NAcGM3-VSSP) or of immunization 4 (aluminum phosphate ). The results are shown in Table 23. As can be seen, both antigenic preparations resulted in an induction of specific titers for VEGF-A, those induced with PVM-I being higher in both adjuvants. These titers were correlated, in turn, with increases in the neutralizing capacity of the sera.

**Table 23. Analysis of the humoral response in non-human primates.**

| | **Treatment Group** | | | |
|---|---|---|---|---|
| **Animal** | **PVM in Alum Phosphate** | **PVM-I in Alum Phosphate** | **PVM in NAcGM3-VSSP** | **PVM-I in NAcGM3-VSSP** |
| 1 | 17830.9 | 54315.64 | 2776.882 | 5591.977 |
| 2 | 24673.76 | 45220.69 | 2585.966 | 5126.05 |
| 3 | 25177.13 | 32738.94 | 2729.897 | 6105.441 |
| 4 | 10060.54 | 25681.82 | 2804.34 | 4878.99 |
| Average | 19436 | 39489 | 2724 | 5426 |
| Standard Deviation | 7092 | 12766 | 97 | 541 |
| Mann-Whitney | | p= **0.0286** | | p= **0.0286** |

| | | | | |
|---|---|---|---|---|
| *Note: Specific titers for human VEGF-A using two adjuvant choices are shown.* | | | | |

To evaluate the extent to which activation of the cellular response was achieved, parallel to the induction of the humoral response, a conventional Delayed-Type Hypersensitivity (DTH) test was performed, as described (Morera, et al., Vaccine, 2010: 28: 3453-61). At 48 h, two perpendicular measurements of induration with digital Vernier calipers were made. The area of the lesions was calculated, and their geometric mean was reported. Erythema or inflammation was not considered part of this reaction. A diameter of 0.5 mm was considered as the limit of a detectable reaction. The results were represented according to the following qualification: (++)> 5 mm²; (+) = between 0.5 and 4.99 mm²; (-) reaction not detectable. The results of such scoring are shown in Table 24. Besides, 6 mm² punches (6 per animal) were made at the sensitization points, which were analyzed by hematoxylin/eosin staining studies. At least two sections from each biopsy were analyzed, to determine the nature of the infiltrate, in terms of the presence of mononuclear cells, neutrophils, or eosinophils.

Intradermal injection of the antigen was well tolerated, with no adverse events such as blisters or ulcers. The monkeys of all groups treated with the vaccine preparations, in their different combinations with adjuvant, reacted against human VEGF-A. In the sites where the saline solution was injected, as a control, no reaction was reported in any of the immunized groups. Histopathological evaluation corroborated the existence of a strong DTH-type reaction to VEGF-A inoculation. The biopsies obtained from the injection sites with human VEGF-A were consistent with a DTH scene, with abundant infiltration of macrophages and lymphocytes.

**Table 24. The score of the DTH reaction in non-human primates immunized with PVM or PVM-I in the presence of two adjuvants.**

| **Experimental Groups** | **Challenge** | |
|---|---|---|
| | Saline Solution | hVEGF-A121 |
| 1. 400 µg PVM in NAcGM3-VSSP | - | (++) |
| 2. 400 µg PVM-I in NAcGM3-VSSP | - | (++) |
| 3. Placebo in NAcGM3-VSSP | - | - |
| 4. 400µg PVM in Alum phosphate | - | (++) |
| 5. 400µg PVM-I in Alum phosphate | - | (++) |
| 6. Placebo in Alum phosphate | - | - |

| | | |
|---|---|---|
| *Six sites were analyzed per animal.* -, *No detectable reaction.* | | |

Peripheral Blood Mononuclear Cells (PBMC) from immunized animals were isolated by Ficoll gradient, as previously reported, and were analyzed for specific cytolysis of syngeneic cells previously incubated with VM, and marked with CFSE. Table 25 shows the results corresponding to the analysis, by flow cytometry, of the survival of the cells "charged" and labeled with CFSE. The analysis of at least three replicates per animal indicates that in the week after the last immunization, corresponding to the induction period, the vaccine preparation induces a specific cellular immunity for the antigen, with cytotoxic capacity. As can be seen, in both adjuvants, the effect of the vaccine was significantly greater when using the PVM-I antigen (P <0.05; Dunnet).

**Table 25. Direct cytolysis of VEGF-A "charged" autologous PBMC isolated from monkeys immunized with PVM or PVM-I in two adjuvants.**

| **Adjuvant** | ***Experimental Groups*** | ***% Cytotoxicity*** | | | |
|---|---|---|---|---|---|
| | | **Animal 1** | **Animal 2** | **Animal 3** | **Animal 4** |
| NAc-GM3-VSSP | I-400 µg PVM | 23 | 24 | 35 | 40 |
| | II-400 µg PVM-I | 48 | 50 | 57 | 46 |
| | III-Adjuvant | 27 | 0 | 0 | 0 |
| Alum Phosphate | IV-400 µg PVM | 29 | 18 | 27 | 27 |
| | V-400 µg PVM-I | 43 | 26 | 38 | 38 |
| | VI-Adjuvant | 10 | 0 | 7 | 0 |

| | | | | | |
|---|---|---|---|---|---|
| *Cytotoxicity was evaluated using Flow cytometry and expressed as the percentage in the reduction of the population tagged with CFSE, as compared to "no charged" autologous PBMCs.* | | | | | |

In these animals, the prevention of experimental laser-induced choroidal neovascularization in non-human primates was also evaluated. As a model, the one reported by Krzystolik et al. (Krzystolik M.G., et al. 2006. Acta Ophthalmol, 120: 338-346) was used. 4 monkeys were used per experimental group. Animals were anesthetized for all procedures, with intramuscular injections of ketamine hydrochlorate, acepromazine maleate, and atropine sulfate. Topical proparacaine hydrochlorate anesthesia was also used. One week after the last immunization, the formation of choroidal neovascularization (CNV) was induced in the macula by argon laser burns. Photography and fluorescent angiography were used to detect and measure the extent, as well as to evaluate the characteristics of the lesions. The development of CNV lesions was evaluated before and after laser treatment, and on days 15, 20, and 29 after it. The lesions were analyzed by a specialist outside the experimental design, using the following scale: Grade 1, no hyper-fluorescence; grade 2, hyper-fluorescence without effusion; grade 3 early or mid-transit hyper-fluorescence and late leakage; and grade 4, very bright early or mid-transit hyper-fluorescence, with late leaks that extend beyond the edges of the laser spot. Daily observations of the animals were made to evaluate their clinical status, including any ocular abnormalities.

Of the four grades assigned to laser treatment, grade 4 corresponds clinically to significant leak status. The lesions are considered to reflect the presence of new choroidal vessels that have either grown beyond the laser treatment area or are leaking so intensely that the fluorescein has noticeably spread beyond the vessels. The mean number of grade 4 lesions in the placebo group ranged from 45.4% to 50.2% of the laser treatment areas. The mean percentage of grade 4 lesions in the control groups was similar to that reported by other authors who have used this animal model of CNV. In contrast, all groups treated with the vaccine preparations showed a marked reduction, or the complete absence of grade 4 lesions, regardless of the adjuvant used. Table 26 shows the percentage distribution of all degrees of injury, on day 29, for the treatment groups. It is interesting that with the use of the vaccine preparation PVM-I, a markedly greater reduction of the lesions of higher gradation is obtained (ANOVA, Bonferroni a posteriori test, p <0.05).

**Table 26. Analysis of the degree of choroidal vascularization.**

| **Groups** | **LESIONS GRADE** | | | |
|---|---|---|---|---|
| | **1** | **2** | **3** | **4** |
| | | | | |
| PVM in sNAcGM3-VSSP | *70.9* | *21.0* | 3.2 | 5.9 |
| PVM-I in sNAcGM3-VSSP | 84.4 | 15.6 | 0.0 | 0.0 |
| Placebo in sNAcGM3-VSSP | 25.8 | 23.1 | 5.7 | 45.4 |
| PVM in Alum Phosphate | 69.8 | 19.2 | 5.2 | *5.8* |
| PVM-I in Alum Phosphate | 83.4 | 15.8 | *0.8* | 0.0 |
| Placebo in Alum Phosphate | 26.2 | 21.1 | 2.5 | 50.2 |

### Example 14. Treatment of solid tumors in humans by the administration of the vaccine composed of the antigenic preparation PVM-I and adjuvants.

This study aimed to evaluate the potential of the new antigenic preparation to induce a relevant specific immune response, in the context of tumor pathology in humans. Patients with solid tumors in advanced stages, with no other therapeutic option, with a period free from other treatments of at least 4 weeks before the start of the administrations were chosen. The antigenic preparation PVM-I was administered in doses of 800 µg: (a) in the presence of sNAcGM3-VSSP, in a weekly inoculation scheme, for 8 weeks, followed by administrations once a month, or (b) in phosphate of Aluminum, in a 4-dose schedule with a bi-weekly frequency, also followed by monthly administrations.

The humoral and cellular immune response was evaluated in the serum, and in the PBMC collected in both cases before the start of the immunization schedules and a week after the last immunization. Two fundamental parameters were evaluated in the serum: the specific antibody titer for VEGF-A and their ability to neutralize its binding to its VEGFR2 receptor. This parameter was determined using two ELISA-type systems (Morera, et al., Vaccine, 2012: 30: 368-77, Sanchez Ramirez, et al., J Immunoassay Immunochem, 2016: 37: 636-58). A titer greater than 1:500, and a neutralization percentage greater than 10% was defined as a positive result.

The cellular response was evaluated in the PBMC samples by IFN-gamma ELISPOT, as described (Gavilondo, et al., Vaccine, 2014: 32: 2241-50). A low responder is defined as a patient with a number of clones positive for IFN-gamma secretion, between 29 and 39 per million CD3 positive cells; a medium responder, one with a number of signals between 40 and 79; and high responders showed more than 80 signals.

The administration of PVM-I in both adjuvants resulted in the establishment of a specific immune response for VEGF-A, in terms of specific antibodies (Figure 4A) and their ability to neutralize the interaction of the growth factor with the VEGFR2 (Figure 4B). This response was observed in 70% of the patients treated with any of the adjuvants used. Similarly, a significant increase in the responsiveness of PBMC to stimulation with a variant of human VEG-A (VM) was observed (Figure 4C). These results indicated that the use in humans of the new antigenic variant PVM-I induces a specific immune response against human VEGF-A.

Figure 5 shows, as a summary, the number of total patients and how many of them showed a positive response in any of the tests for each of the adjuvants. The results were stratified, taking into account the number of tests with a significantly higher result when using their pre-immune values as a control. In the graph, each patient is also assigned the number of months that survived after the start of administration of the vaccine preparations (analysis at 24 months). This makes it possible to relate to what extent the specific immune response contributes to an increase in survival, in the absence of another onco-specific treatment. It is observed that in patients with a higher number of positive responses, survival increased significantly (p <0.05; Kaplan Meyer).

### Example 15. Clinical use of the antigenic preparation PVM-I in the treatment of age-related macular degeneration in humans.

This study aimed to evaluate the potentiality of the antigenic preparation PVM-I to induce a relevant specific immune response in the context of Age-related Macular Degeneration (AMD) in humans. The patients received administrations of Bevacizumab, at a rate of 125 µg in 50 µL, by the intravitreal route, once a month, for three consecutive months. The administration of this drug or other antiangiogenic drugs such as Aflibercept, Ranibizumab, or Ramucirumab for the first three months after diagnosis, is what has been established as conventional therapy for the initial control of this disease. After these administrations, the patient systematically attends visual acuity checks and phenotypic variations in the structure of the retinal membranes and, depending on the changes in these, new intravitreal injections of antiangiogenics are indicated or not. The success of the treatment is largely determined by the reduction in the need for intravitreal administrations.

This therapy was administered concomitantly, or not, with the antigenic preparation PVM-I, in doses of 400 µg: (Group 2) in the presence of sNAcGM3-VSSP on a once a week schedule, for 8 weeks, and then re-administration was performed once a month, or (Group 3) in Aluminum Phosphate in a 4-dose schedule with a bi-weekly frequency, followed also by administrations once a month. Group 1 only received conventional therapy consisting of the administration of a monthly dose for 3 consecutive months. After the first three administrations of Bevacizumab, the patients only received them again in case of worsening of the lesions when compared with the previous ophthalmological analysis. This aggravation was defined as a loss in visual acuity, an increase in retinal thickness, the presence of intraretinal fluid, or an increase in subretinal fluid (above 200 µm or the value observed in the previous visit).

Administration of the antigenic preparation in the indicated adjuvants and regimens resulted in a significant reduction in the number of intravitreal injections of Bevacizumab required to maintain or improve the quality of the patient's vision and the integrity of the macula. Table 27 shows, as a summary, the number of Bevacizumab injections per patient that it was necessary to apply after the third administration of the same, and the specific antibody titers for VEGF-A that induces immunization. The use of immunotherapy with the antigenic preparation PVM-I significantly reduces (p<0.05 in both cases, Dunnet's test), the number of intravitreal injections of Bevacizumab to be applied to patients compared to the conventional scheme (Group 1). In both cases, a significant correlation was observed between the reduction in the number of intravitreal administrations and the level of specific antibodies for human VEGF-A in the serum of the patients, one week after the fourth immunization for group 2 (Pearson r=-0.8788, p=0.0008) and from the eighth immunization for group 3 (Pearson r=-0.7894, p=0.0066).

**Table 27. Evaluations of patients in the study**

| **Group 1** | | **Group 2** | | | **Group 3** | | |
|---|---|---|---|---|---|---|---|
| **Patient Code** | **A** | **Patient Code** | **A** | **B** | **Patient Code** | **A** | **B** |
| **FP01** | 2 | **FP03** | 2 | 5560 | **FP07** | 0 | 15987 |
| **FP02** | 8 | **FP04** | 3 | 4875 | **FP09** | 0 | 24920 |
| **FP05** | 5 | **FP08** | 4 | 2871 | **FP10** | 1 | 3852 |
| **FP06** | 2 | **FP14** | 1 | 3952 | **FP11** | 6 | 1360 |
| **FP12** | 7 | **FP15** | 0 | 10250 | **FP17** | 6 | 1350 |
| **FP13** | 7 | **FP16** | 2 | 5023 | **FP19** | 4 | 2800 |
| **FP18** | 7 | **FP20** | 5 | 995 | **FP23** | 2 | 4600 |
| **FP22** | 8 | **FP21** | 4 | 1885 | **FP25** | 4 | 2750 |
| **FP24** | 6 | **FP26** | 2 | 5587 | **FP28** | 5 | 1900 |
| **FP30** | 5 | **FP27** | 6 | 1190 | **FP29** | 5 | 1530 |

| | | | | | | | |
|---|---|---|---|---|---|---|---|
| ***A:** Number of intravitreal injections needed and, **B:** VEGF-A specific antibody titers generated after the antigen administration, **Group 1:** Conventional therapy, **Group 2:** 400 µg de PVM-I in Alum Phosphate, **Group 3:** 400 µg of PVM-I in sNAcGM3-VSSP.* | | | | | | | |

Approximately 30% of the patients included in the study had active extrafoveal polyps. This is a poor prognostic factor for the evolution of patients in response to conventional intravitreal administration therapy. This is because polypoid lesions originate in deeper layers of the retina. Interestingly, the analysis of the patients who presented with choroidal polypoidal neovasculature indicated a significant reduction in the number of active lesions, one year after initiating therapy, an effect that was only described for 20% of patients (1 of 5) in the group to which the conventional therapy was administered (Table 28).

**Table 28. Evaluation of extrafoveal polypoid lesions in study patients.**

| **Patient Code** | **Treatment Group** | **Number of active polyps/number at the treatment initiation** | **Número de pólipos activos/total al año de tratamiento** |
|---|---|---|---|
| **PF12** | I | 4/4 | 6/6 |
| **PF18** | I | 3/3 | 4/3 |
| **PF24** | I | 2/2 | 3/2 |
| **PF20** | II | 3/3 | 0/0 |
| **PF21** | II | 4/2 | 0/0 |
| **PF27** | II | 3/2 | 1/1 |
| **PF11** | III | 3/3 | 1/0 |
| **PF17** | III | 3/2 | 1/0 |
| **PF28** | III | 4/2 | 2/1 |
| **PF29** | III | 2/2 | 0/0 |

## Claims

1. A polypeptide **characterized by** comprising a functional mutated form of an isoform of human vascular endothelial growth factor A, (VEGF-A) that fold in a non-natural arrangement of disulfide bridges where the second and fourth cysteines of the polypeptide chain of the mutant are only found as part of intramolecular bridges, and the seventh and eighth cysteines of the mutant are only found forming intermolecular bonds.

2. The polypeptide of claim 1 **characterized in that** the functional mutant is obtained from the mutation of the amino acids of VEGF-A involved in binding to its type 2 receptor (VEGFR2).

3. The polypeptide of claim 2 **characterized in that** the human VEGF-A isoform is selected from the group consisting of VEGF-A¹²¹, VEGF-A¹⁴⁵, VEGF-A¹⁶⁵, VEGF-A¹⁸⁹, and VEGF-A²⁰⁶.

4. The polypeptide of claim 3 **characterized in that** it comprises a rearrangement of intramolecular disulfide bridges where the first cysteine is linked to the second cysteine, the third cysteine to the fourth cysteine, the fifth cysteine with the sixth cysteine; and where the seventh cysteine of two polypeptide chains, the eighth cysteine of two polypeptide chains and the last cysteine of two polypeptide chains are linked forming intermolecular disulfide bridges.

5. The polypeptide from claim 3 **characterized in that** it comprises an intramolecular disulfide bridge rearrangement of intramolecular disulfide bridges where the first cysteine is linked to the second cysteine, the third cysteine to the fourth cysteine, the fifth cysteine with the sixth cysteine; and where the seventh cysteine of a polypeptide chain with the eighth cysteine of another polypeptide chain, and the last cysteine of two polypeptide chains, are linked forming intermolecular disulfide bridges.

6. The polypeptide from claim 3 **characterized in that** it comprises an intramolecular disulfide bridge rearrangement of intramolecular disulfide bridges where the first cysteine is linked to the second cysteine, the third cysteine to the fifth cysteine, fourth cysteine with the sixth cysteine; and where the seventh cysteine of two polypeptide chains, the eighth cysteine of two polypeptide chains and the last cysteine of two polypeptide chains are linked forming intermolecular disulfide bridges.

7. The polypeptide from claim 3 **characterized in that** it comprises an intramolecular disulfide bridge rearrangement of intramolecular disulfide bridges where the first cysteine is linked to the second cysteine, the third cysteine to the fifth cysteine, fourth cysteine with the sixth cysteine; and where the seventh cysteine of a polypeptide chain with the eighth cysteine of another polypeptide chain, and the last cysteine of two polypeptide chains, are linked forming intermolecular disulfide bridges.

8. The polypeptide from claim 3 **characterized in that** it comprises an intramolecular disulfide bridge rearrangement of intramolecular disulfide bridges where the first cysteine is linked to the second cysteine, the third cysteine to the sixth cysteine, fourth cysteine with the fifth cysteine; and where the seventh cysteine of two polypeptide chains, the eighth cysteine of two polypeptide chains and the last cysteine of two polypeptide chains are linked forming intermolecular disulfide bridges.

9. The polypeptide from claim 3 **characterized by** comprising an intramolecular disulfide bridge rearrangement of intramolecular disulfide bridges where the first cysteine is linked to the second cysteine, the third cysteine to the fourth cysteine, the fifth cysteine with the sixth cysteine; and where the seventh cysteine of a polypeptide chain with the eighth cysteine of another polypeptide chain, and the last cysteine of two polypeptide chains, are linked forming intermolecular disulfide bridges.

10. The polypeptide from claim 3 **characterized by** comprising an intramolecular disulfide bridge rearrangement of intramolecular disulfide bridges where the first cysteine is linked to the last cysteine, the second cysteine to the third cysteine, the fourth cysteine with the fifth cysteine; and where the seventh cysteine of two polypeptide chains, and the eighth cysteine of two polypeptide chains, are linked forming intermolecular disulfide bridges.

11. The polypeptide from claim 3 **characterized by** comprising an intramolecular disulfide bridge rearrangement of intramolecular disulfide bridges where the first cysteine is linked to the last cysteine, the second cysteine to the third cysteine, the fourth cysteine with the fifth cysteine, and where the seventh cysteine of a polypeptide chain with the eighth cysteine of another polypeptide chain are linked forming intermolecular disulfide bridges.

12. The polypeptide of claim 3 **characterized in that** it comprises a rearrangement of intramolecular disulfide bridges where the first cysteine is linked to the last cysteine, the second cysteine to the fourth cysteine, the third cysteine with the fifth cysteine; and where the seventh cysteine of two polypeptide chains, and the eighth cysteine of two polypeptide chains, are linked forming intermolecular disulfide bridges.

13. The polypeptide of claim 3 **characterized in that** it comprises a rearrangement of intramolecular disulfide bridges where the first cysteine is linked to the last cysteine, the second cysteine to the fourth cysteine, the third cysteine with the fifth cysteine; and where the seventh cysteine of a polypeptide chain with the eighth cysteine of another polypeptide chain are linked by forming intermolecular disulfide bridges.

14. The polypeptide of claim 3 **characterized in that** it comprises a rearrangement of intramolecular disulfide bridges where the first cysteine is linked to the last cysteine, the second cysteine to the fifth cysteine, the third cysteine with the fourth cysteine; and where the seventh cysteine of two polypeptide chains, and the eighth cysteine of two polypeptide chains, are linked forming intermolecular disulfide bridges.

15. The polypeptide of claim 3 **characterized in that** it comprises a rearrangement of intramolecular disulfide bridges where the first cysteine is linked to the last cysteine, the second cysteine to the fifth cysteine, the third cysteine with the fourth cysteine; and where the seventh cysteine of a polypeptide chain with the eighth cysteine of another polypeptide chain are linked by forming intermolecular disulfide bridges.

16. The polypeptides of any of claims 4-15 comprising an amino-terminal segment to increase its expression in bacteria and a carboxi terminal segment that facilitates purification.

17. The polypeptide of claim 16 wherein the amino-terminal segment to increase its expression in bacteria has an amino acid sequence consisting of SEQ ID NO: 24.

18. The polypeptide of claim 17 **characterized in that** its amino acid sequence is selected from the group consisting of the sequences of SEQ ID NO: 18 to SEQ ID NO: 23.

19. An antigenic preparation comprising at least one polypeptide of any of claims 4-18 and a pharmaceutically acceptable excipient or diluent.

20. A pharmaceutical composition comprising the antigenic preparation of claim 19 and a pharmaceutically accepted vaccine adjuvant.

21. The pharmaceutical composition of claim 20 wherein the vaccine adjuvant is selected from the group consisting of oil adjuvants, aluminum salts, proteoliposomes, and proteoliposomes conjugated to gangliosides.

22. Use of the antigenic preparation comprising at least one polypeptide of any of claims 4-18 for the manufacture of a medicament for the treatment of diseases whose progress is related to the increase in angiogenesis, inflammation, and immunosuppression.

23. The use of claim 22 wherein the disease whose progression is related to increased angiogenesis, inflammation, and immunosuppression is selected from the group consisting of cancer, macular degeneration, diabetes, rheumatoid arthritis, and edema.

24. Use of the antigenic preparation comprising at least one polypeptide of any of claims 4-18 for the manufacture of a medicament for the restoration of the immune system.
